# EUROPÄISCHE PATENTSCHRIFT

(11) **EP 2 456 780 B1**
(45) Veröffentlichungstag und Bekanntmachung des Hinweises auf die Patenterteilung: **20.09.2017**
(21) Anmeldenummer: 10730103.8
(22) Anmeldetag: 08.07.2010
(51) Int. Cl.: C07J 7/00

(54) **17-HYDROXY-17-PENTAFLUORETHYL-ESTRA-4,9(10)-DIEN-11-ARYL-DERIVATE, VERFAHREN ZU IHRER HERSTELLUNG UND IHRE VERWENDUNG ZUR BEHANDLUNG VON KRANKHEITEN**
17-HYDROXY-17-PENTAFLUORETHYL-ESTRA-4,9(10)-DIEN-11-ARYL DERIVATIVES, METHODS FOR THE PRODUCTION THEREOF AND THE USE THEREOF FOR TREATING DISEASES
DÉRIVÉS 17-HYDROXY-17-PENTAFLUOROÉTHYL-ESTRA-4,9(10)-DIÈNE-11-ARYLE, PROCÉDÉS DE PRÉPARATION ET UTILISATION POUR LE TRAITEMENT DE MALADIES

(30) Priorität: 21.07.2009 DE 102009034525
(43) Veröffentlichungstag der Anmeldung: 30.05.2012
(73) Patentinhaber: Bayer Intellectual Property GmbH, 40789 Monheim (DE)
(72) Erfinder: KLAR, Ulrich, 13503 Berlin (DE); SCHWEDE, Wolfgang, 16548 Glienicke (DE); MÖLLER, Carsten, 10115 Berlin (DE); ROTGERI, Andrea, 13503 Berlin (DE); BONE, Wilhelm, 13467 Berlin (DE)
(74) Vertreter: BIP Patents
(86) Internationale Anmeldenummer: PCT/EP2010/004157
(87) Internationale Veröffentlichungsnummer: WO 2011/009534

(56) Entgegenhaltungen:
- WO-A1-98/34947
- DE-A1-102006 054 535

## Beschreibung

Die Erfindung betrifft 17-Hydroxy-17-pentafluorethyl-estra-4,9(10)-dien-11-aryl-Derivate der Formel II und III mit Progesteron antagonisierender Wirkung und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose können die erfindungsgemäßen Verbindungen auch sequentiell in Kombination mit Gestagenen verabreicht werden. In einem solchen Behandlungsregime könnten die erfindungsgemäßen Verbindungen über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.

Die Wirksamkeit der erfindungsgemäßen Verbindungen als Progesteronrezeptorantagonist wurde in vitro in Transaktivierungstests gezeigt. Verbindungen mit antagonistischer Wirkung am Progesteronrezeptor (kompetitive Progesteronrezeptorantagonisten) sind erstmals 1982 bekannt (RU 486; EP57115) und seither zahlreich beschrieben worden. Progesteronrezeptorantagonisten mit fluorierter 17α-Seitenkette wurden in WO 98/34947 und Fuhrmann et al., J. Med. Chem. 43, 5010 - 5016 (2000) veröffentlicht.

Die in WO 98/34947 beschriebenen Verbindungen mit fluorierter 17α-Seitenkette weisen im allgemeinen eine sehr starke antagonistische Aktivität am Progesteronrezeptor auf. Sehr potente und daher in WO 98/34947 bevorzugte Verbindungen sind 11β-(4-Acetylphenyl)-20,20,21,21,21-pentafluor-17-hydroxy-19-nor-17α-pregna-4,9-dien-3-on, 11β-(4-Acetylphenyl)-20,20,21,21,21-pentafluor-17-hydroxy-19-nor-17α-pregna-4-en-3-on und 6'-Acetyl-9,11β-dihydro-17β-hydroxy-17α-(1,1,2,2,2-pentafluorethyl)-4'H-naphth[3',2',1':10,9,11]ester-4-en-3-on. Diese Verbindungen werden in vivo in erheblichem Maße in verschiedene Metabolite überführt, die zum Teil starke, zum Teil geringere pharmakologische Aktivität aufweisen. Der Metabolismus tritt überwiegend am 4-Substituenten des 11-Phenylrestes auf. In WO2008/058767 werden Verbindungen beschrieben, die zumindest zum Teil Metabolite der in WO 98/34947 beschriebenen Verbindungen sind.

Aufgabe der vorliegenden Erfindung ist es, hoch potente kompetitive Progesteronrezeptorantagonisten zur Verfügung zu stellen und damit alternative Behandlungsmöglichkeiten gynäkologischer Erkrankungen zu schaffen.

Es wurde gefunden, dass die erfindungsgemäßen Verbindungen besonders geeignet sind um diese Aufgabe zu lösen.

Der Pfeil in den oben angeführten Teilstrukturen bedeutet die Anknüpfungsstelle an Struktur nach Formel II und III.

Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.

Jeder der genannten Substituenten am Steroid-Grundgerüst kann sowohl in einer α-als auch in einer β-Stellung vorliegen. Außerdem können auch die Substituenten am Steroid-Grundgerüst, die eine Doppelbindung enthalten und in denen die Doppelbindung an jedem Atom mindestens einen Substituenten, der nicht Wasserstoff ist, trägt, sowohl E- als auch Z-konfiguriert vorliegen.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methyl-aminomethan und 1-Amino-2,3,4-butantriol. Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche während ihrer Verweilzeit im Körper zu erfindungsgemäßen Verbindungen beispielsweise durch enzymatische oder hydrolytische Prozesse umgesetzt werden.

Im Rahmen der vorliegenden Erfindung haben die Substituenten, soweit nicht anders spezifiziert, die folgende Bedeutung:
Alkyl steht für gerad- oder verzweigtkettige Alkylgruppen mit 1-6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl Hexyl, Heptyl, Octyl, Nonyl und Decyl.
Aryl steht für einen mono- bis tricyclischen aromatischen, substituierten oder unsubstituierten carbocyclischen Rest, wie zum Beispiel Phenyl, Naphthyl, die einfach oder mehrfach mit Halogen (F, Cl, Br, I), OH, O-Alkyl, CO₂H, CO₂-Alkyl, NH₂, NH(C₁-C₁₀-Alkyl), N(C₁-C₁₀-Alkyl)₂, insbesondere N(CH₃)₂, NO₂, N₃, CN, C₁-C₁₀-Alkyl, C₁-C₁₀-Perfluor-Alkyl, C₁-C₁₀-Acyl, C₁-C₁₀-Acyloxy-Gruppen, substituiert sein können.
Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Benzofuranyl, Benzothiophenyl, Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl, Tetrazolyl.
Aralkyl steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl, Furylmethyl, Thienylethyl, Pyridylpropyl in Betracht. Die Ringe können einfach oder mehrfach durch Halogen, OH, O-Alkyl, CO₂H, CO₂-Alkyl, NH₂, NH(C₁-C₁₀-Alkyl), N(C₁-C₁₀-Alkyl)₂, NO₂, N₃, CN, C₁-C₂₀-Alkyl, C₁-C₁₀-Perfluor-Alkyl, C₁-C₂₀-Acyl, C₁-C₂₀-Acyloxy-Gruppen substituiert sein.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Die vorliegende Erfindung betrifft die Verbindungen der Formel (II), 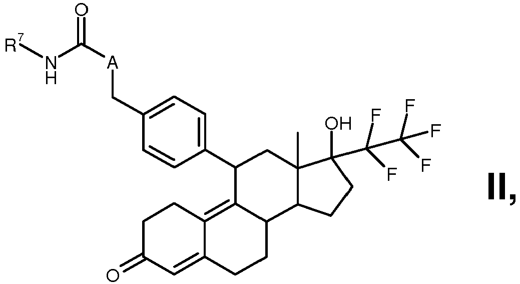 worin
- A: entweder -O- oder -NH-,
- R⁷: C₁-C₄-Alkyl-, Allyl-, gegebenenfalls para- Postion durch die Gruppen C₁-C₄-Alkyl, -CN, C₁-C₄-Alkoxy, Halogen, C₁-C₄-Alkanoyloxy, -CO₂H, -CO₂C₁₋₄-Alkyl oder Piperidinyl substituiertes Phenyl-, 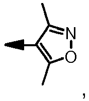 Benzyl-, -(CH₂)ₘ-COOR⁸ mit m = 1, 2 oder 3 und R⁸ = Wasserstoff oder C₁-C₄-Alkyl bedeutet.

Die Verbindungen der Formel (III) 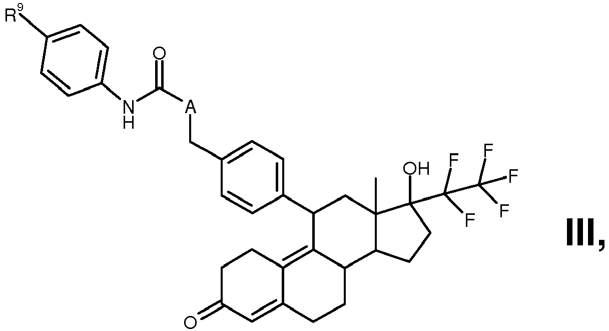 worin
- A: entweder -O- oder -NH- und
- R⁹: Wasserstoff, C₁-C₄-Alkyl, -CN, C₁-C₄-Alkoxy, Halogen, C₁-C₄-Alkanoyloxy, - CO₂H, oder -CO₂C₁₋₄-Alkyl
bedeutet.
- 1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-*1H-*cyclopenta[a]phenanthren-11-yl)-benzyl]-harnstoff (Beispiel 17)
- 1-Allyl-3-[4-((8S,11R,13S,14S,17S)-17-hydroxy-13-methyl-3-oxo-17-pentafluorethyl-2,3,6,7,8,11,12,13,14,15,16,17-dodecahydro-*1H-*cyclopenta[a]phenanthren-11-yl)-benzyl]-harnstoff (Beispiel 18)
- 1-Isopropyl-3-[4-((8S,11R,13S,14S,17S)-17-hydroxy-13-methyl-3-oxo-17-pentafluorethyl-2,3,6,7,8,11,12,13,14,15,16,17-dodecahydro-*1H-*cyclopenta[a]phenanthren-11-yl)-benzyl]-harnstoff (Beispiel 19)
- 1-*tert*-Butyl-3-[4-((8S,11R,13S, 14S,17S)-17-hydroxy-13-methyl-3-oxo-17-pentafluorethyl-2,3,6,7,8,11,12,13,14,15,16,17-dodecahydro-*1H-*cyclopenta[a]phenanthren-11-yl)-benzyl]-harnstoff (Beispiel 20)
- {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzyl]-ureido}-essigsäureethylester (Beispiel 21)
- 3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzyl]-ureido}-propionsäureethylester (Beispiel 22)
- 1-Benzyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 23)
- 1-Phenyl-3-[4-((8S,11R,13S,14S,17S)-1-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 24)
- {3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-ureido}-essigsäure (Beispiel 26)
- 3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-*cy*clopenta[*a*]phenanthren-11-yl)-benzyl]-ureido}-propionsäure (Beispiel 27)
- 1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-3-(4-methoxy-phenyl)-harnstoff (Beispiel 32)
- 1-(4-Fluor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 33)
- 1-(4-Chlor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 34)
- 1-(4-*tert-*Butyl-phenyl)-3-[4-((8S,11R,13S,14S, 17S)-17-hydroxy-13-methyl-3-oxo-17-pentafluorethyl-2,3,6,7,8,11,12,13,14,15, 16,17-dodecahydro-1*H-*cyclopenta[*a*]phenanthren-11-yl)-benzyl]-harnstoff (Beispiel 35)
- 4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-ureido}-benzoesäure ethylester (Beispiel 36)
- 4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-ureido}-benzoesäure (Beispiel 38)
- 1-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-3-(4-piperidin-1-yl-phenyl)-harnstoff (Beispiel 39)
- Ethyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzylester (Beispiel 41)
- Isopropyl-carbaminsäure 4-((8S,11R,13S,14S, 17S)-17-hydroxy-13-methyl-3-oxo-17-pentafluorethyl-2,3,6,7,8,11,12,13,14,15, 16,17-dodecahydro-1*H-*cyclopenta[*a*]phenanthren-11-yl)-benzylester (Beispiel 42)
- Allyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzylester (Beispiel 43)
- *tert*-Butyl-carbaminsäure 4-((8S,11R,13S,14S, 17S)-17-hydroxy-13-methyl-3-oxo-17-pentafluorethyl-2,3,6,7,8,11,12,13,14,15,16, 17-dodecahydro-1*H-*cyclopenta[*a*]phenanthren-11-yl)-benzylester (Beispiel 44)
- (4-Piperidin-1-yl-phenyl)-carbaminsäure 4-((8S,11R,13S,14S,17S)-17-hydroxy-13-methyl-3-oxo-17-pentafluorethyl-2,3,6,7,8,11,12,13, 14,15,16,17-*dodecahydro*-1*H-*cyclopenta[*a*] phenanthren-11-yl)-benzylester (Beispiel 45)
- Phenyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1*H*-cyclopenta[*a*] phenanthren-11-yl)-benzylester (Beispiel 46)
- (4-Methoxy-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 47)
- (4-Methyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 48)
- (4-Fluor-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 49)
- (4-Chlor-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 50)
- (4-*tert*-Butyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 51)
- 4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyloxycarbonylamino]-benzoesäure ethylester (Beispiel 52)
- [4-(3,5-Dimethyl-isoxazol-4-yl)-phenyl]-carbaminsäure 4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzylester (Beispiel 53)
- 3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyloxycarbonylamino]-propionsäure (Beispiel 55)
- Benzyl-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H*-cyclopenta[*a*] phenanthren-11-yl)-benzylester (Beispiel 56)
- 3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyloxycarbonylamino]-propionsäureethylester (Beispiel 57)

Einige der hier genannten hoch potenten Progesteronrezeptorantagonisten besitzen eine für den Menschen vorhergesagte orale Bioverfügbarkeit von < 50%, weshalb sie sich besonders gut für lokale Anwendungen, die eine reduzierte systemische Stabilität erfordern, eignen. Dazu gehören beispielweise die in den Beispielen 17, 23, 24 und 41 genannten Verbindungen (siehe Tabellen 1 und 2).

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Es wurde gefunden, dass die erfindungsgemäßen Verbindungen bzw. Derivate eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose verursachten Symptome (insbesondere Schmerzen) deutlich reduziert werden können.

Die erfindungsgemäßen Verbindungen zeigen ein nicht vorhersehbares, wertvolles pharmakologisches, pharmakokinetisches und pharmakodynamisches Wirkprofil.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Progesteronrezeptorantagonisten, also ihre antagonisierende Wirkung am Progesteronrezeptor erklären.

Weiterer Gegenstand der vorliegenden Erfindung ist der Einsatz der erfindungsgemäßen Verbindungen zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Uterusfibroiden, der Endometriose und von hormonabhängigen Mammakarzinomen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung von 0,1-100 mg der erfindungsgemäßen Verbindungen pro Tag und Patientin bei der Behandlung von Uterusfibroiden oder der Endometriose und für die kontrazeptive Anwendung bzw. von 0,1-500 mg der erfindungsgemäßen Verbindungen pro Tag und Patientin bei Tumor-Erkrankungen (z.B. Menginiome oder hormonabhängige Tumore wie z.B. das Mammakarzinom) und bei der Notfallkontrazeption.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika.

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonists gefolgt von der 14tägigen Gabe des Gestagens.

Zur Behandlung von mit der Menopause assoziierten Beschwerden kommt eine gleichzeitige oder sequentielle Verabreichung der erfindungsemäßen Verbindungen z.B. mit SERMs, SERDs und Östrogenen in Betracht.

SERMs (Selective Estrogen Receptor Modulators) sind erfindungsgemäß solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen. Selektive Estrogenrezeptordestabilisatoren (SERD) sind Arzneistoffe, die den Estrogenrezeptor antagonisieren (,reine Antiöstrogene' ohne östrogene Wirkkomponente) und zu einem Abbau des Rezeptors führen (beispielsweise Fulvestrant, ZK-703 und ZK-253 [Hoffmann J et al., J Natl Cancer Inst 2004, 96:210-218] sowie in WO 98/007740, WO 99/33855 und WO 03/045972 beschriebene Verbindungen.

Antiöstrogene sind Verbindungen die den Estrogenrezeptor antagonisieren, beispielsweise Fulvestrant.

Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.

Kinaseinhibitoren hemmen Enzyme, die einen Phosphatrest von ATP auf andere Substrate, dort insbesondere auf Hydroxygruppen, übertragen, z.B. Sorafenib (Nexavar) oder Imatinib (Gleevec).

Angiogenesehemmer, z.B. Avastin, reduzieren bzw. blockieren die Gefäßversorgung und damit die Durchblutung eines Tumors.

Zytostatika, z.B. cis-Platin, Taxol, Taxotere, Sagopilone, Ixabepilone sind natürliche oder synthetische Substanzen, die das Zellwachstum bzw. die Zellteilung hemmen.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die erfindungsgemäßen Verbindungen können systemisch und/oder lokal wirken. Zu diesem Zweck können sie auf geeignete Weise, wie z.B. oral, intrauterin[är], intravaginal, parenteral, pulmonal, nasal, sublingual, lingual, buccal, rectal, dermal, transdermal, conjunctival, optisch oder als Implantat bzw. Stent appliziert werden. Intrauterin[är] bedeutet dabei insbesondere die Applikation mittels IUS (intrauterine system) oder IUD (intrauterine device). Die intravaginale Applikation kann u.a. mittels IVR/VRS (Intra-Vaginalring/Vaginalringsystem) erfolgen.

Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nonsilikon- und/oder Silikonpolymere, insbesondere auch siloxanebasierte Elastomere (vergl. WO 01/47490, insbesondere Seite 7, Zeile 19 - Seite 15, Zeile 15), enthalten. Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele dienen der Erläuterung der Erfindung ohne diese in irgend einer Weise zu beschränken.

### Beispiel 17

### 1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

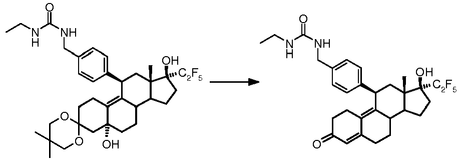

In Analogie zu Beispiel 1 setzte man 44 mg (max. 67µmol) der nach Beispiel 17a dargestellten Verbindung um und isolierte nach Aufarbeitung und Reinigung 16 mg (43%) der Titelverbindung als farblosen Schaum.
¹H-NMR (CDCl₃): δ= 0,57 (3H), 1,07 (3H), 1,36-1,56 (2H), 1,71-1,89 (3H), 2,05 (1H), 2,14-2,64 (9H), 2,70 (1H), 3,09-3,24 (3H), 4,20 (1H), 4,29 (1H), 4,40 (1H), 4,60 (1H), 4,91 (1H), 5,75 (1H), 7,10 (2H), 7,15 (2H) ppm.

### Beispiel 17a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-ethyl-harnstoff

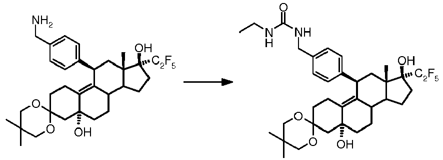

Die Lösung von 40 mg (67 µmol) der nach Beispiel 17b dargestellten Verbindung in 0,5 ml Dichlormethan versetzte man mit 5,5 µl Ethylisocyanat und rührte 1 Stunde bei 23°C. Man engte ein und setzte das erhaltene Rohpro dukt ohne Reinigung weiter um.

### Beispiel 17b

### (5R,8S,11R,13S,14S,17S)-11-(4-Aminomethyl-phenyl)-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-5,17-diol

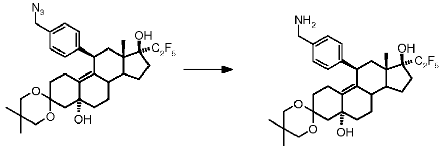

Die Lösung von 1,8 g (2,88 mmol) der nach Beispiel 17c dargestellten Verbindung in 50 ml Tetrahydrofuran versetzte man mit 8 ml Wasser, 0,56 ml Trimethylphosphin und rührte 4 Stunden bei 23°C. Man versetzte mit 6 ml einer 25%igen Ammoniaklösung, rührte weitere 16 Stunden bei 23°C und engte ein. Die als Rohprodukt erhaltene Titelverbindung setzte man ohne Reinigung weiter um.

### Beispiel 17c

### (5R,8S,11R,13S,14S,17S)-11-(4-Azidomethyl-phenyl)-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan-5,17-diol

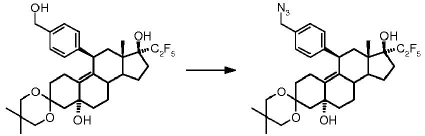

Die Lösung von 2,0 g (3,33 mmol) der nach Beispiel 17d dargestellten Verbindung in 50 ml Tetrahydrofuran versetzte man bei 3°C mit 1 ml Diphenylphosphorylazid, 0,58 ml 1,8-Diazabicyclo[5.4.0]undec-7-en und ließ 4 Stunden bei 23°C sowie weitere 16 Stunden bei 60°C reagieren. Man versetzte mit Wasser, extrahierte mehrfach mit Ethylacetat, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 1,85 g (89%) der Titelverbindung als farbloser Schaum.

### Beispiel 17d

### (5R,8S,11R,13S,14S,17S)-11-(4-Hydroxymethyl-phenyl)-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan-5,17-diol

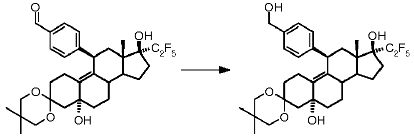

Die Lösung von 2,0 g (3,34 mmol) der nach Beispiel 2c dargestellten Verbindung in 12 ml Tetrahydrofuran und 1,2 ml Methanol versetzte man bei 3°C mit 70 mg Natriumborhydrid und rührte 2,5 Stunden. Man goss in gesättigte Ammoniumchloridlösung, extrahierte mehrfach mit Ethylacetat, wusch die vereinigten organischen Extrakte mit gesättigter Natriumchloridlösung und trocknete über Natriumsulfat. Nach Filtration und Lösungsmittelabzug isolierte man 2,0 g (100%) der Titelverbindung als farbloser Schaum, die man ohne Reinigung weiter umsetzte.

### Beispiel 18

### 1-Allyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

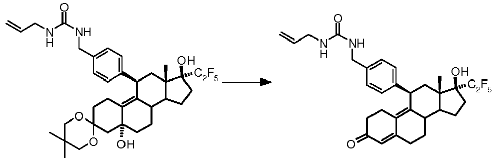

In Analogie zu Beispiel 1 setzte das nach Beispiel 18a dargestellte Rohprodukt um und isolierte nach Aufarbeitung und Reinigung 18,6 mg (48%) der Titelverbindung als farblosen Schaum.
¹H-NMR (CDCl₃): δ= 0,58 (3H), 1,37-1,56 (2H), 1,71-1,88 (3H), 2,05 (1H), 2,16-2,64 (9H), 2,65-2,77 (2H), 3,78 (2H), 4,22-4,37 (2H), 4,42 (1H), 4,61 (1H), 4,86 (1H), 5,08 (1H), 5,14 (1H), 5,76 (1H), 5,82 (1H), 7,11 (2H), 7,17 (2H) ppm.

### Beispiel 18a

### 1-Allyl-3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzyl]-harnstoff

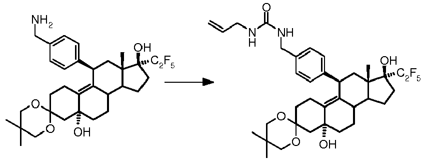

In Analogie zu Beispiel 17a setzte man 40 mg (67 µmol) der nach Beispiel 17b dargestellten Verbindung unter Verwendung von Allylisocyanat um. Das nach Aufarbeitung isolierte Rohprodukt wurde ohne Reinigung weiter umgesetzt.

### Beispiel 19

### 1-Isopropyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

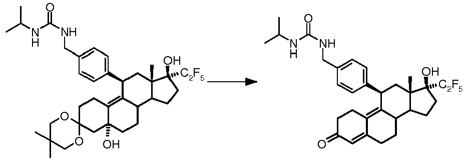

In Analogie zu Beispiel 1 setzte man das nach Beispiel 19a dargestellte Rohprodukt um und isolierte nach Aufarbeitung und Reinigung 16,9 mg (43%) der Titelverbindung als farblosen Schaum.
¹H-NMR (CDCl₃): δ= 0,58 (3H), 1,11 (6H), 1,37-1,56 (2H), 1,72-1,88 (3H), 2,05 (1H), 2,16-2,64 (9H), 2,71 (1H), 2,81 (1H), 3,83 (1H), 4,19-4,36 (3H), 4,41 (1H), 4,69 (1H), 5,76 (1H), 7,11 (2H), 7,17 (2H) ppm.

### Beispiel 19a

### 1-lsopropyl-3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzyl]-harnstoff

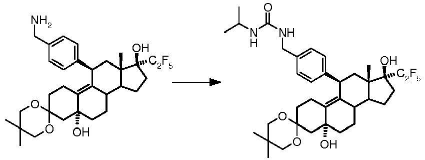

In Analogie zu Beispiel 17a setzte man 40 mg (67 µmol) der nach Beispiel 17b dargestellten Verbindung unter Verwendung von Isopropylisocyanat um. Das nach Aufarbeitung isolierte Rohprodukt wurde ohne Reinigung weiter umgesetzt.

### Beispiel 20

### 1-tert-Butyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

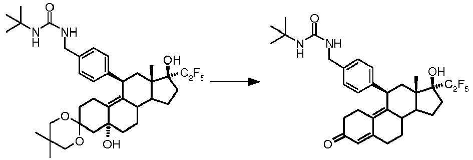

In Analogie zu Beispiel 1 setzte man das nach Beispiel 20a dargestellte Rohprodukt um und isolierte nach Aufarbeitung und Reinigung 18,4 mg (46%) der Titelverbindung als farblosen Schaum.
¹H-NMR (CDCl₃): δ= 0,58 (3H), 1,31 (9H), 1,39-1,56 (2H), 1,72-1,87 (3H), 2,05 (1H), 2,17-2,63 (10H), 2,71 (1H), 4,18-4,34 (3H), 4,42 (1H), 4,53 (1H), 5,77 (1H), 7,11 (2H), 7,18 (2H) ppm.

### Beispiel 20a

### 1-tert-Butyl-3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzyl]-harnstoff

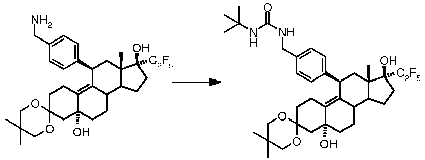

In Analogie zu Beispiel 17a setzte man 40 mg (67 µmol) der nach Beispiel 17b dargestellten Verbindung unter Verwendung von *tert*-Butylisocyanat um. Das nach Aufarbeitung isolierte Rohprodukt wurde ohne Reinigung weiter umgesetzt.

### Beispiel 21

### {3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-essigsäureethylester

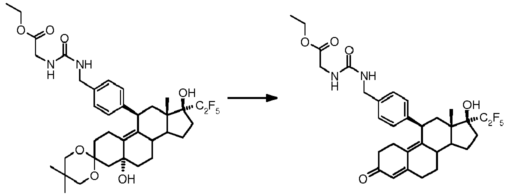

In Analogie zu Beispiel 1 setzte man das nach Beispiel 21a dargestellte Rohprodukt um und isolierte nach Aufarbeitung und Reinigung 16,7 mg (40%) der Titelverbindung als farblosen Schaum.
¹H-NMR (CDCl₃): δ= 0,57 (3H), 1,26 (3H), 1,37-1,55 (2H), 1,71-1,87 (3H), 2,05 (1H), 2,17-2,63 (9H), 2,66 (1H), 2,71 (1H), 3,90-4,06 (3H), 4,13-4,24 (2H), 4,31 (1H), 4,42 (1H), 5,06 (2H), 5,77 (1H), 7,11 (2H), 7,18 (2H) ppm.

### Beispiel 21a

### {3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-essigsäureethylester

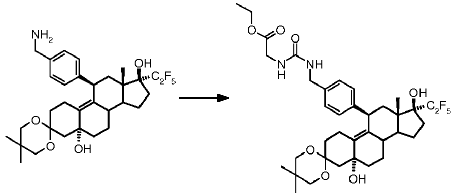

In Analogie zu Beispiel 17a setzte man 40 mg (67 µmol) der nach Beispiel 17b dargestellten Verbindung unter Verwendung von Isocyanatoessigsäureethylester um. Das nach Aufarbeitung isolierte Rohprodukt wurde ohne Reinigung weiter umgesetzt.

### Beispiel 22

### 3-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-propionsäureethylester

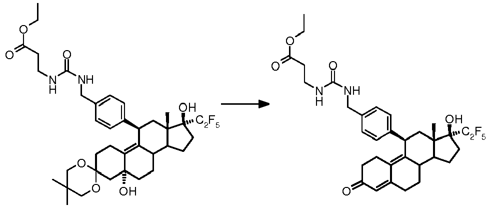

In Analogie zu Beispiel 1 setzte man das nach Beispiel 22a dargestellte Rohprodukt um und isolierte nach Aufarbeitung und Reinigung 16,4 mg (38%) der Titelverbindung als farblosen Schaum.
¹H-NMR (CDCl₃): δ= 0,58 (3H), 1,25 (3H), 1,38-1,56 (2H), 1,71-1,88 (3H), 2,05 (1H), 2,17-2,65 (11H), 2,71 (1H), 2,74 (1H), 3,44 (2H), 4,09 (1H), 4,14 (1H), 4,20-4,35 (2H), 4,42 (1H), 4,82 (1H), 5,05 (1H), 5,77 (1H), 7,11 (2H), 7,17 (2H) ppm.

### Beispiel 22a

### 3-{3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-propionsäureethylester

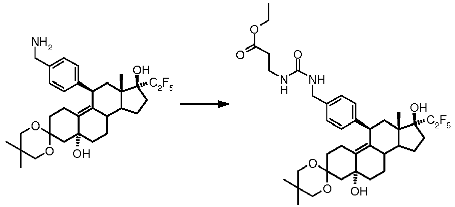

In Analogie zu Beispiel 17a setzte man 40 mg (67µmol) der nach Beispiel 17b dargestellten Verbindung unter Verwendung von 3-Isocyanatopropionsäureethylester um. Das nach Aufarbeitung isolierte Rohprodukt wurde ohne Reinigung weiter umgesetzt.

### Beispiel 23

### 1-Benzyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

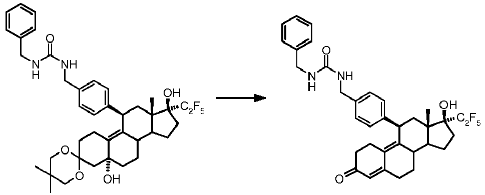

In Analogie zu Beispiel 1 setzte man das nach Beispiel 23a dargestellte Rohprodukt um und isolierte nach Aufarbeitung und Reinigung 19,1 mg (45%) der Titelverbindung als farblosen Schaum.
¹H-NMR (CDCl₃): δ= 0,56 (3H), 1,38-1,55 (2H), 1,71-1,87 (3H), 2,05 (1H), 2,13-2,63 (10H), 2,69 (1H), 4,21-4,36 (4H), 4,40 (1H), 4,80-4,89 (2H), 5,75 (1H), 7,09 (2H), 7,14 (2H), 7,20-7,33 (5H) ppm.

### Beispiel 23a

### 1-Benzyl-3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyciopenta[a]phenanthren-3,2'-[1,3]dioxan]-11-yl)-benzyl]-harnstoff

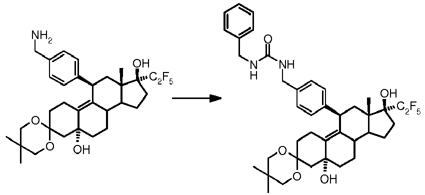

In Analogie zu Beispiel 17a setzte man 40 mg (67µmol) der nach Beispiel 17b dargestellten Verbindung unter Verwendung von Benzylisocyanat um. Das nach Aufarbeitung isolierte Rohprodukt wurde ohne Reinigung weiter umgesetzt.

### Beispiel 24

### 1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-3-phenyl-harnstoff

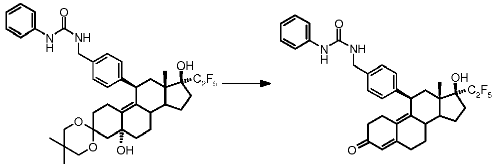

In Analogie zu Beispiel 1 setzte man das nach Beispiel 24a dargestellte Rohprodukt um und isolierte nach Aufarbeitung und Reinigung 18,7 mg (46%) der Titelverbindung als farblosen Schaum.
¹H-NMR (CDCl₃): δ= 0,54 (3H), 1,37-1,54 (2H), 1,70-1,87 (3H), 2,03 (1H), 2,11-2,61 (9H), 2,66 (1H), 2,82 (1H), 4,20-4,43 (3H), 5,58 (1H), 5,75 (1H), 7,00 (1H), 7,07 (2H), 7,14 (2H), 7,21-7,30 (5H) ppm.

### Beispiel 24a

### 1-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-phenyl-harnstoff

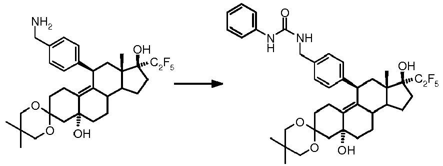

In Analogie zu Beispiel 17a setzte man 40 mg (67 µmol) der nach Beispiel 17b dargestellten Verbindung unter Verwendung von Phenylisocyanat um. Das nach Aufarbeitung isolierte Rohprodukt wurde ohne Reinigung weiter umgesetzt.

### Beispiel 26

### {3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-essigsäure

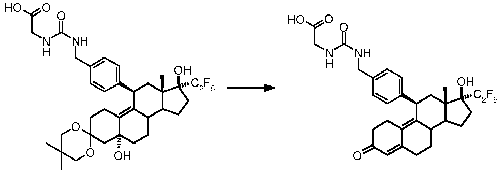

In Analogie zu Beispiel 1 setzte man 58 mg (83 µmol) der nach Beispiel 26a dargestellten Verbindung um und isolierte nach Aufarbeitung und Reinigung 22 mg (45%) der Titelverbindung als farblosen Schaum.
¹H-NMR (CD₃OD): δ= 0,57 (3H), 1,36-1,54 (2H), 1,70-1,82 (3H), 2,09 (1H), 2,16-2,46 (5H), 2,55-2,72 (4H), 2,79 (1H), 3,70 (2H), 4,26 (2H), 4,50 (1H), 5,73 (1H), 7,17 (2H), 7,22 (2H) ppm.

### Beispiel 26a

### {3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-essigsäure

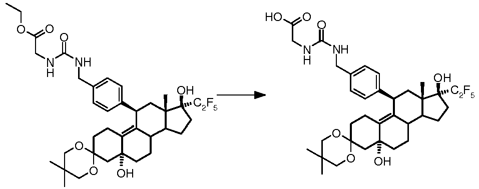

Die Lösung von 85 mg (120 µmol) der nach Beispiel 21a dargestellten Verbindung in einem Gemisch aus 1 ml Tetrahydrofuran und 1 ml Ethanol versetzte man mit 1 ml einer 5%igen wäßrigen Lithiumhydroxidlösung und rührte 3 Stunden bei 23°C. Man verdünnte mit Wasser, säuerte durch Zugabe einer 1 molaren Salzsäure an, sättigte mit Natriumchlorid, extrahierte mehrfach mit Diethylether und trocknete die vereinigten organischen Extrakte über Natriumsulfat. Nach Filtration und Lösungsmittelabzug isolierte man 64 mg (78%) der Titelverbindung als farblosen Schaum.

### Beispiel 27

### 3-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-propionsäure

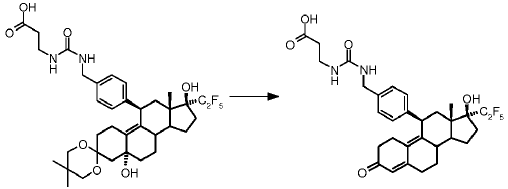

In Analogie zu Beispiel 1 setzte man 62 mg (87 µmol) der nach Beispiel 27a dargestellten Verbindung um und isolierte nach Aufarbeitung und Reinigung 22 mg (42%) der Titelverbindung als farblosen Schaum.
¹H-NMR (CD₃OD): δ= 0,57 (3H), 1,36-1,54 (2H), 1,70-1,82 (3H), 2,09 (1H), 2,16-2,45 (7H), 2,55-2,71 (4H), 2,79 (1H), 3,35 (2H), 4,24 (2H), 4,49 (1H), 5,73 (1H), 7,18 (4H) ppm.

### Beispiel 27a

### 3-{3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-propionsäure

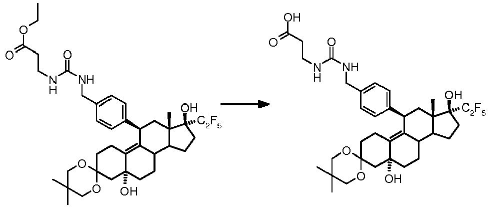

In Analogie zu Beispiel 26a setzte man 87 mg (120 µmol) der nach Beispiel 22a dargestellten Verbindung um und isolierte nach Aufarbeitung 68 mg (81%) der Titelverbindung als farblosen Schaum.

### Beispiel 32

### 1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-3-(4-methoxy-phenyl)-harnstoff

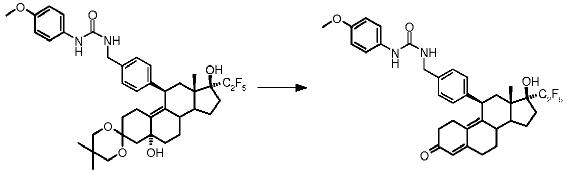

In Analogie zu Beispiel 1 setzte man 50 mg (67 µmol) der nach Beispiel 32a dargestellten Verbindung um und isolierte nach Aufarbeitung und Reinigung 21 mg (49%) der Titelverbindung als farblosen Schaum.
¹H-NMR (CDCl₃): δ= 0,56 (3H), 1,37-1,54 (2H), 1,72-1,86 (3H), 2,04 (1H), 2,15-2,62 (9H), 2,66 (1H), 2,70 (1H), 3,77 (3H), 4,34 (2H), 4,40 (1H), 5,19 (1H), 5,76 (1H), 6,49 (1H), 6,82 (2H), 7,06-7,19 (6H) ppm.

### Beispiel 32a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-(4-methoxy-phenyl)-harnstoff

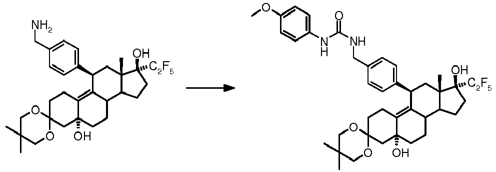

Die Lösung aus 40 mg (67 µmol) der nach Beispiel 17b dargestellten Verbindung in 1 ml Dichlormethan versetzte man mit 9 µl 4-Methoxyphenylisocyanat und rührte 2 Stunden bei 23°C. Man engte ein und setzte den erhaltenen Rückstand ohne Reinigung weiter um.

### Beispiel 33

### 1-(4-Fluor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

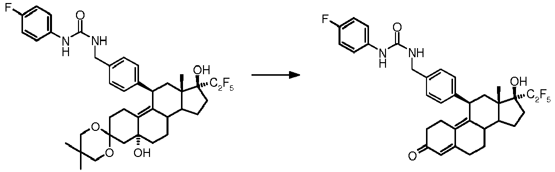

In Analogie zu Beispiel 1 setzte man 49 mg (67 µmol) der nach Beispiel 33a dargestellten Verbindung um und isolierte nach Aufarbeitung und Reinigung 21 mg (50%) der Titelverbindung als farblosen Schaum.
¹H-NMR (CDCl₃): δ= 0,54 (3H), 1,36-1,54 (2H), 1,71-1,88 (3H), 2,04 (1H), 2,10-2,72 (10H), 2,88 (1H), 4,27 (2H), 4,39 (1H), 5,59 (1H), 5,75 (1H), 6,90 (2H), 7,02-7,24 (7H) ppm.

### Beispiel 33a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-(4-fluor-phenyl)-harnstoff

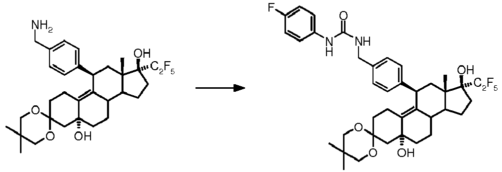

In Analogie zu Beispiel 32a setzte man 40 mg (67 µmol) der nach Beispiel 17b dargestellten Verbindung unter Verwendung von 4-Fluorphenylisocyanat um und isolierte nach Aufarbeitung 50 mg der Titelverbindung als Rohprodukt.

### Beispiel 34

### 1-(4-Chlor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

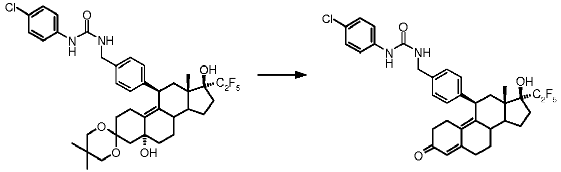

In Analogie zu Beispiel 1 setzte man 50 mg (66 µmol) der nach Beispiel 34a dargestellten Verbindung um und isolierte nach Aufarbeitung und Reinigung 21 mg (50%) der Titelverbindung als farblosen Schaum.
¹H-NMR (CDCl₃): δ= 0,51 (3H), 1,34-1,51 (2H), 1,67-1,82 (3H), 2,01 (1H), 2,10-2,61 (11H), 2,67 (1H), 4,30 (2H), 4,37 (1H), 5,73 (1H), 7,02-7,29 (9H) ppm.

### Beispiel 34a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-(4-chlor-phenyl)-harnstoff

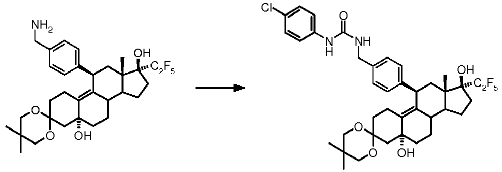

In Analogie zu Beispiel 32a setzte man 40 mg (67 µmol) der nach Beispiel 17b dargestellten Verbindung unter Verwendung von 4-CHlorphenylisocyanat um und isolierte nach Aufarbeitung 51 mg der Titelverbindung als Rohprodukt.

### Beispiel 35

### 1-(4-tert-Butyl-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff

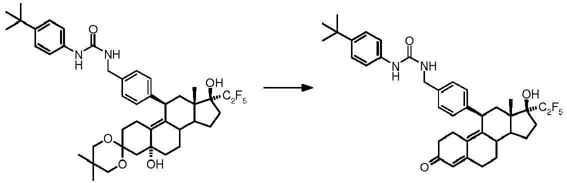

In Analogie zu Beispiel 1 setzte man 51 mg (66 µmol) der nach Beispiel 35a dargestellten Verbindung um und isolierte nach Aufarbeitung und Reinigung 20,5 mg (46%) der Titelverbindung als farblosen Schaum.
¹H-NMR (CDCl₃): δ= 0,56 (3H), 1,28 (9H), 1,37-1,55 (2H), 1,71-1,87 (3H), 2,05 (1H), 2,15-2,62 (9H), 2,65 (1H), 2,69 (1H), 4,33 (2H), 4,40 (1H), 5,37 (1H), 5,76 (1H), 6,64 (1H), 7,09 (2H), 7,13-7,21 (4H), 7,30 (2H) ppm.

### Beispiel 35a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-(4-tert.-butyl-phenyl)-harnstoff

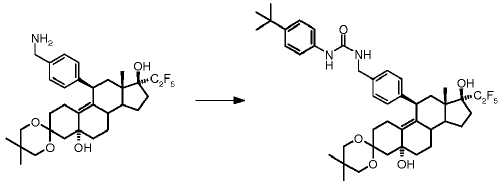

In Analogie zu Beispiel 32a setzte man 40 mg (67 µmol) der nach Beispiel 17b dargestellten Verbindung unter Verwendung von 4-*tert*-Butylphenylisocyanat um und isolierte nach Aufarbeitung 52 mg der Titelverbindung als Rohprodukt.

### Beispiel 36

### 4-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-benzoesäure ethylester

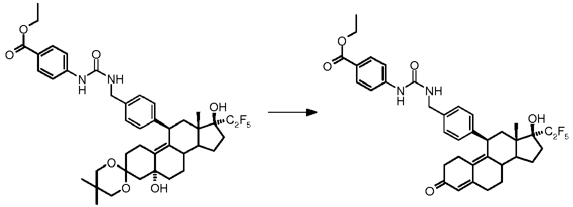

In Analogie zu Beispiel 1 setzte man 67 mg (85 µmol) der nach Beispiel 36a dargestellten Verbindung um und isolierte nach Aufarbeitung und Reinigung 21 mg (37%) der Titelverbindung als farblosen Schaum.
¹H-NMR (CDCl₃): δ= 0,58 (3H), 1,36 (3H), 1,39-1,54 (2H), 1,71-1,87 (3H), 1,98-2,22 (3H), 2,27-2,70 (8H), 3,25 (1H), 4,27-4,42 (5H), 5,75 (1H), 6,08 (1H), 7,08 (2H), 7,15 (2H), 7,36 (2H), 7,84 (2H), 7,90 (1H) ppm.

### Beispiel 36a

### {3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-4-benzoesäureethylester

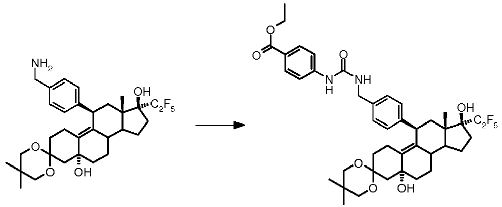

In Analogie zu Beispiel 32a setzte man 100 mg (0,17 mmol) der nach Beispiel 17b dargestellten Verbindung unter Verwendung von 4-Isocyanatbenzoesäureethylester um und isolierte nach Aufarbeitung 137 mg der Titelverbindung als Rohprodukt.

### Beispiel 38

### 4-{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-benzoesäure

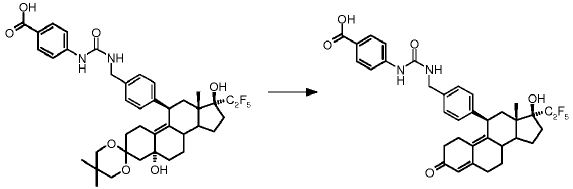

In Analogie zu Beispiel 1 setzte man 71 mg (93 µmol) der nach Beispiel 38a dargestellten Verbindung um und isolierte nach Aufarbeitung und Reinigung 15 mg (26%) der Titelverbindung als farblosen Schaum.
¹H-NMR (CD₃OD): δ= 0,57 (3H), 1,25-1,56 (2H), 1,68-1,83 (3H), 2,02-2,46 (6H), 2,53-2,72 (4H), 2,79 (1H), 4,34 (2H), 4,50 (1H), 5,72 (1H), 7,20 (2H), 7,25 (2H), 7,41 (2H), 7,87 (2H) ppm.

### Beispiel 38a

### {3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-ureido}-4-benzoesäure

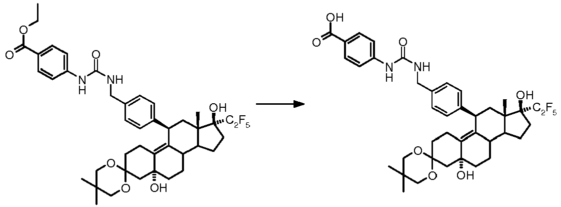

In Analogie zu Beispiel 26a setzte man 69 mg (87 µmol) der nach Beispiel 36a dargestellten Verbindung um und isolierte nach Aufarbeitung 71 mg der Titelverbindung als Rohprodukt.

### Beispiel 39

### 1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-3-(4-piperidin-1-yl-phenyl)-harnstoff

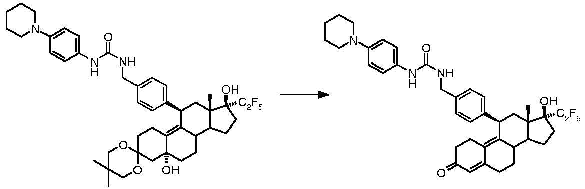

In Analogie zu Beispiel 1 setzte man 27 mg (34 µmol) der nach Beispiel 39a dargestellten Verbindung um und isolierte nach Aufarbeitung und Reinigung 9,8 mg (42%) der Titelverbindung als farblosen Schaum.
¹H-NMR (CDCl₃): δ= 0,55 (3H), 1,37-1,86 (11H), 2,04 (1H), 2,16-2,62 (9H), 2,70 (1H), 2,79 (1H), 3,10 (4H), 4,33 (2H), 4,40 (1H), 5,15 (1H), 5,76 (1H), 6,37 (1H), 6,86 (2H), 7,05-7,17 (6H) ppm.

### Beispiel 39a

### 1-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyl]-3-(4-piperidin-1-yl-phenyl)-harnstof

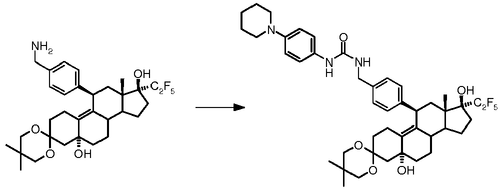

In Analogie zu Beispiel 32a setzte man 40 mg (67 µmol) der nach Beispiel 17b dargestellten Verbindung unter Verwendung von 4-Piperidinphenylisocyanat um und isolierte nach Aufarbeitung 55 mg der Titelverbindung als Rohprodukt.

### Beispiel 41

### Ethyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzylester

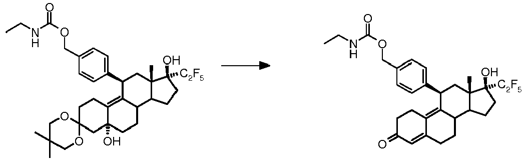

In Analogie zu Beispiel 1 setzte man das in Beispiel 41a dargestellte Rohprodukt um und isolierte nach Aufarbeitung und Reinigung 19 mg (50%) der Titelverbindung als farblosen Schaum.
¹H-NMR (CDCl₃): δ= 0,60 (3H), 1,13 (3H), 1,39-1,57 (2H), 1,73-1,87 (3H), 2,06 (1H), 2,19-2,64 (10H), 2,72 (1H), 3,23 (2H), 4,44 (1H), 4,71 (1H), 5,03 (2H), 5,78 (1H), 7,16 (2H), 7,25 (2H) ppm.

### Beispiel 41a

### Ethyl-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

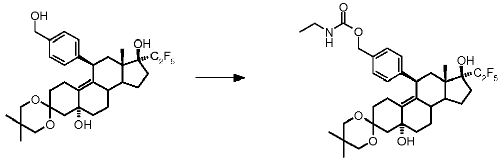

In Analogie zu Beispiel 32a setzte man 40 mg (67µmol) der nach Beispiel 17d dargestellten Verbindung unter Verwendung von Ethylisocyanat um und setzte die nach Aufarbeitung erhaltene Titelverbindung als Rohprodukt weiter um.

### Beispiel 42

### Isopropyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzylester

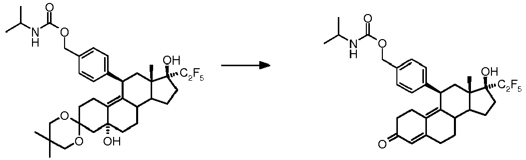

In Analogie zu Beispiel 1 setzte man das in Beispiel 42a dargestellte Rohprodukt um und isolierte nach Aufarbeitung und Reinigung 22,3mg (58%) der Titelverbindung als farblosen Schaum.
¹H-NMR (CDCl₃): δ= 0,60 (3H), 1,15 (6H), 1,38-1,56 (2H), 1,72-1,87 (3H), 2,06 (1H), 2,20-2,65 (10H), 2,72 (1H), 3,82 (1H), 4,44 (1H), 4,58 (1H), 5,01 (2H), 5,78 (1H), 7,16 (2H), 7,25 (2H) ppm.

### Beispiel 42a

### Isopropyl-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

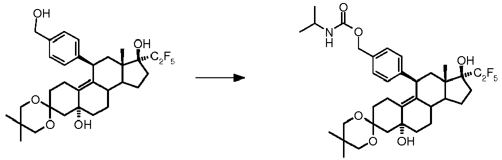

In Analogie zu Beispiel 32a setzte man 40 mg (67 µmol) der nach Beispiel 17d dargestellten Verbindung unter Verwendung von Isopropylisocyanat um und setzte die nach Aufarbeitung erhaltene Titelverbindung als Rohprodukt weiter um.

### Beispiel 43

### Allyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzylester

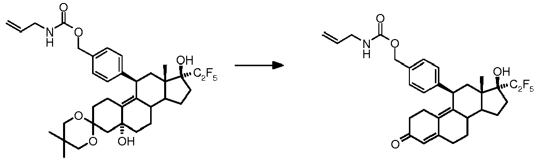

In Analogie zu Beispiel 1 setzte man das in Beispiel 43a dargestellte Rohprodukt um und isolierte nach Aufarbeitung und Reinigung 25 mg (63%) der Titelverbindung als farblosen Schaum.
¹H-NMR (CDCl₃): δ= 0,60 (3H), 1,38-1,56 (2H), 1,72-1,88 (3H), 2,06 (1H), 2,19-2,64 (10H), 2,72 (1H), 3,81 (2H), 4,44 (1H), 4,83 (1H), 5,05 (2H), 5,12 (1H), 5,18 (1H), 5,78 (1H), 5,83 (1H), 7,16 (2H), 7,26 (2H) ppm.

### Beispiel 43a

### Allyl-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

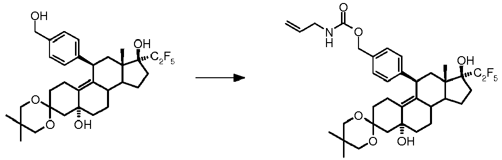

In Analogie zu Beispiel 32a setzte man 40 mg (67 µmol) der nach Beispiel 17d dargestellten Verbindung unter Verwendung von Allylisocyanat um und setzte die nach Aufarbeitung erhaltene Titelverbindung als Rohprodukt weiter um.

### Beispiel 44

### tert-Butyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzylester

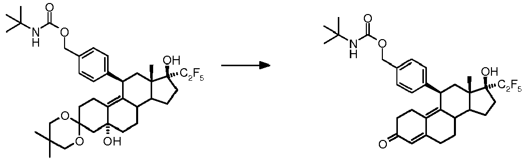

In Analogie zu Beispiel 1 setzte man das in Beispiel 44a dargestellte Rohprodukt um und isolierte nach Aufarbeitung und Reinigung 15 mg (38 %) der Titelverbindung als farblosen Schaum.
¹H-NMR (CDCl₃): δ= 0,59 (3H), 1,32 (9H), 1,40-1,56 (2H), 1,73-1,87 (3H), 2,06 (1H), 2,20-2,64 (10H), 2,72 (1H), 4,44 (1H), 4,71 (1H), 4,99 (2H), 5,78 (1H), 7,16 (2H), 7,25 (2H) ppm.

### Beispiel 44a

### tert-Butyl-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

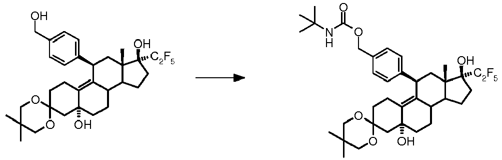

In Analogie zu Beispiel 32a setzte man 40 mg (67 µmol) der nach Beispiel 17d dargestellten Verbindung unter Verwendung von *tert.*-Butylisocyanat um und setzte die nach Aufarbeitung erhaltene Titelverbindung als Rohprodukt weiter um.

### Beispiel 45

### (4-Piperidin-1-yl-phenyl)-carbaminsäure 4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a] phenanthren-11-yl)-benzylester

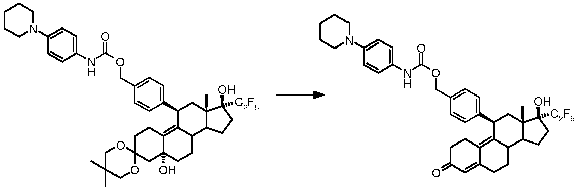

In Analogie zu Beispiel 1 setzte man das in Beispiel 45a dargestellte Rohprodukt um und isolierte nach Aufarbeitung und Reinigung 7,8 mg (17%) der Titelverbindung als farblosen Schaum.
¹H-NMR (CDCl₃): δ= 0,59 (3H), 1,41-1,59 (4H), 1,65-1,85 (7H), 2,06 (1H), 2,12 (1H), 2,22-2,63 (9H), 2,72 (1H), 3,08 (4H), 4,45 (1H), 5,14 (2H), 5,78 (1H), 6,52 (1H), 6,89 (2H), 7,17 (2H), 7,24 (2H), 7,31 (2H) ppm.

### Beispiel 45a

### (4-Piperidin-1-yl-phenyl)--carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

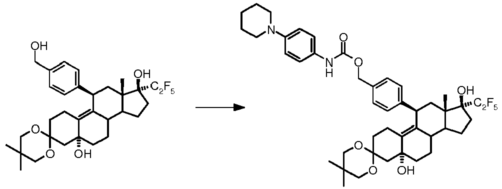

In Analogie zu Beispiel 32a setzte man 40 mg (67 µmol) der nach Beispiel 17d dargestellten Verbindung unter Verwendung von 4-Piperidinphenylisocyanat um und setzte die nach Aufarbeitung erhaltene Titelverbindung als Rohprodukt weiter um.

### Beispiel 46

### Phenyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1H-cyclopenta[a] phenanthren-11-yl)-benzylester

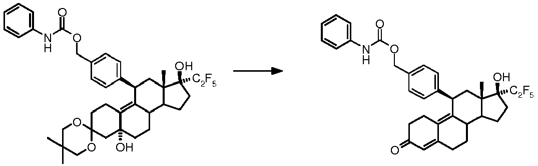

In Analogie zu Beispiel 1 setzte man das in Beispiel 46a dargestellte Rohprodukt um und isolierte nach Aufarbeitung und Reinigung 13 mg (34%) der Titelverbindung als farblosen Schaum.
¹H-NMR (CDCl₃): δ= 0,60 (3H), 1,38-1,56 (2H), 1,73-1,87 (3H), 2,06 (1H), 2,10 (1H), 2,20-2,64 (9H), 2,72 (1H), 4,45 (1H), 5,16 (2H), 5,79 (1H), 6,67 (1H), 7,07 (1H), 7,18 (2H), 7,25-7,40 (6H) ppm.

### Beispiel 46a

### Phenyl-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

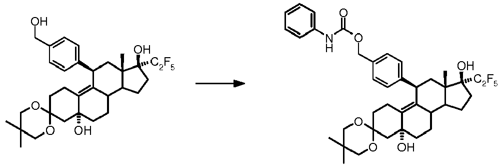

In Analogie zu Beispiel 32a setzte man 40 mg (67 µmol) der nach Beispiel 17d dargestellten Verbindung unter Verwendung von Phenylisocyanat um und setzte die nach Aufarbeitung erhaltene Titelverbindung als Rohprodukt weiter um.

### Beispiel 47

### (4-Methoxy-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester

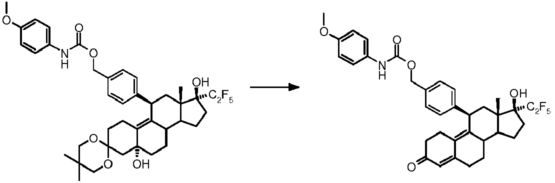

In Analogie zu Beispiel 1 setzte man das in Beispiel 47a dargestellte Rohprodukt um und isolierte nach Aufarbeitung und Reinigung 4,5 mg (10%) der Titelverbindung als farblosen Schaum.
¹H-NMR (CDCl₃): δ= 0,60 (3H), 1,40-1,55 (2H), 1,73-1,87 (3H), 2,02 (1H), 2,06 (1H), 2,21-2,64 (9H), 2,72 (1H), 3,78 (3H), 4,45 (1H), 5,15 (2H), 5,78 (1H), 6,53 (1H), 6,85 (2H), 7,18 (2H), 7,28-7,34 (4H) ppm.

### Beispiel 47a

### (4-Methoxy-phenyl)-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

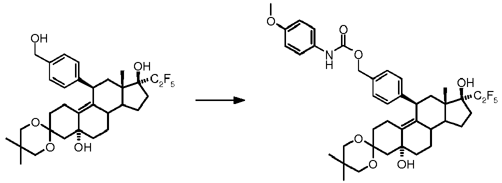

In Analogie zu Beispiel 32a setzte man 40 mg (67 µmol) der nach Beispiel 17d dargestellten Verbindung unter Verwendung von 4-Methoxyphenylisocyanat um und setzte die nach Aufarbeitung erhaltene Titelverbindung als Rohprodukt weiter um.

### Beispiel 48

### (4-Methyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester

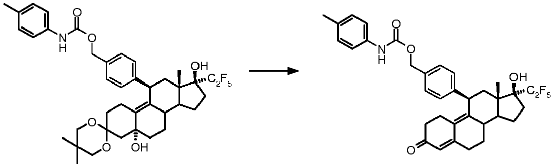

In Analogie zu Beispiel 1 setzte man das in Beispiel 48a dargestellte Rohprodukt um und isolierte nach Aufarbeitung und Reinigung 5,9 mg (14%) der Titelverbindung als farblosen Schaum.
¹H-NMR (CDCl₃): δ= 0,60 (3H), 1,39-1,55 (2H), 1,73-1,87 (3H), 2,04 (1H), 2,07 (1H), 2,20-2,64 (9H), 2,30 (3H), 2,72 (1H), 4,45 (1H), 5,15 (2H), 5,78 (1H), 6,58 (1H), 7,10 (2H), 7,18 (2H), 7,25 (2H), 7,32 (2H) ppm.

### Beispiel 48a

### (4-Methyl-phenyl)-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

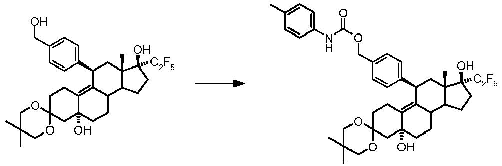

In Analogie zu Beispiel 32a setzte man 40 mg (67 µmol) der nach Beispiel 17d dargestellten Verbindung unter Verwendung von 4-Tolylisocyanat um und setzte die nach Aufarbeitung erhaltene Titelverbindung als Rohprodukt weiter um.

### Beispiel 49

### (4-Fluor-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester

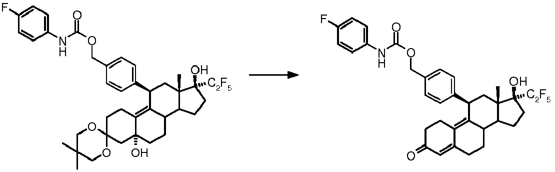

In Analogie zu Beispiel 1 setzte man das in Beispiel 49a dargestellte Rohprodukt um und isolierte nach Aufarbeitung und Reinigung 6 mg (14%) der Titelverbindung als farblosen Schaum.
¹H-NMR (CDCl₃): δ= 0,60 (3H), 1,39-1,56 (2H), 1,73-1,87 (3H), 2,05 (1H), 2,07 (1H), 2,20-2,65 (9H), 2,72 (1H), 4,45 (1H), 5,15 (2H), 5,79 (1H), 6,63 (1H), 7,00 (2H), 7,18 (2H), 7,28-7,34 (4H) ppm.

### Beispiel 49a

### (4-Fluor-phenyl)-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

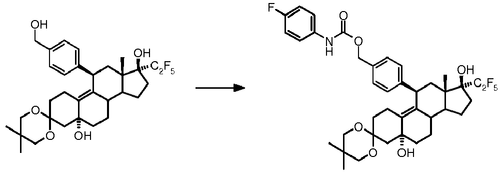

In Analogie zu Beispiel 32a setzte man 40 mg (67 µmol) der nach Beispiel 17d dargestellten Verbindung unter Verwendung von 4-Fluorphenylisocyanat um und setzte die nach Aufarbeitung erhaltene Titelverbindung als Rohprodukt weiter um.

### Beispiel 50

### (4-Chlor-phenyl)-carbaminsäure 4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a] phenanthren-11-yl)-benzylester

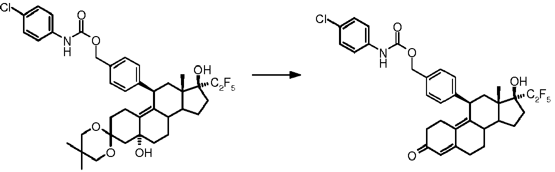

In Analogie zu Beispiel 1 setzte man das in Beispiel 50a dargestellte Rohprodukt um und isolierte nach Aufarbeitung und Reinigung 8,5 mg (20%) der Titelverbindung als farblosen Schaum.
¹H-NMR (CDCl₃): δ= 0,60 (3H), 1,39-1,56 (2H), 1,73-1,87 (3H), 2,06 (1H), 2,07 (1H), 2,19-2,64 (9H), 2,72 (1H), 4,46 (1H), 5,15 (2H), 5,78 (1H), 6,68 (1H), 7,19 (2H), 7,22-7,36 (6H) ppm.

### Beispiel 50a

### (4-Chlor-phenyl)-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

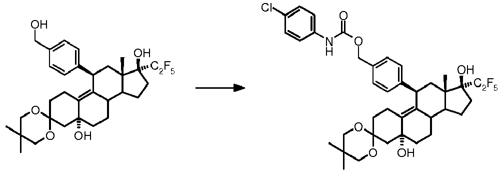

In Analogie zu Beispiel 32a setzte man 40 mg (67 µmol) der nach Beispiel 17d dargestellten Verbindung unter Verwendung von 4-Chlorphenylisocyanat um und setzte die nach Aufarbeitung erhaltene Titelverbindung als Rohprodukt weiter um.

### Beispiel 51

### (4-tert-Butyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester

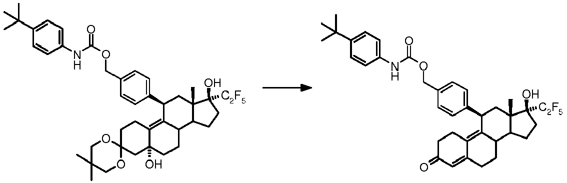

In Analogie zu Beispiel 1 setzte man das in Beispiel 51a dargestellte Rohprodukt um und isolierte nach Aufarbeitung und Reinigung 5,2 mg (12%) der Titelverbindung als farblosen Schaum.
¹H-NMR (CDCl₃): δ= 0,60 (3H), 1,30 (9H), 1,40-1,55 (2H), 1,73-1,87 (3H), 2,02 (1H), 2,06 (1H), 2,21-2,64 (9H), 2,73 (1H), 4,45 (1H), 5,16 (2H), 5,79 (1H), 6,60 (1H), 6,65 (2H), 7,19 (2H), 7,28-7,34 (4H) ppm.

### Beispiel 51a

### (4-tert-Butyl-phenyl)-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydrospiro[cyclopenta[a]phenanthren-3,2'-[1,3]dioxan]-11-yl)-benzylester

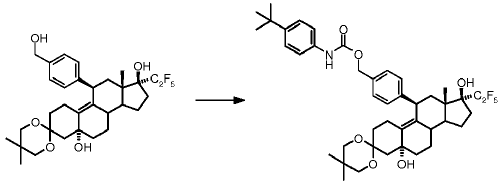

In Analogie zu Beispiel 32a setzte man 40 mg (67 µmol) der nach Beispiel 17d dargestellten Verbindung unter Verwendung von *tert*.-Butylphenylisocyanat um und setzte die nach Aufarbeitung erhaltene Titelverbindung als Rohprodukt weiter um.

### Beispiel 52

### 4-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyloxycarbonylamino]-benzoesäure ethylester

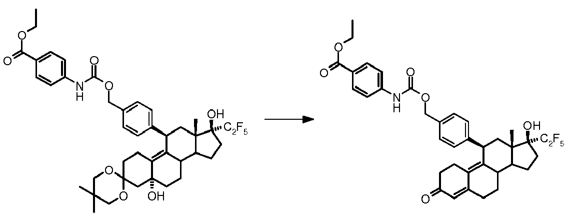

In Analogie zu Beispiel 1 setzte man das in Beispiel 52a dargestellte Rohprodukt um und isolierte nach Aufarbeitung und Reinigung 10 mg (24%) der Titelverbindung als farblosen Schaum.
¹H-NMR (CDCl₃): δ= 0,60 (3H), 1,38 (3H), 1,38-1,55 (2H), 1,74-1,87 (3H), 2,02 (1H), 2,06 (1H), 2,20-2,64 (9H), 2,73 (1H), 4,35 (2H), 4,46 (1H), 5,18 (2H), 5,78 (1H), 6,85 (1H), 7,19 (2H), 7,32 (2H), 7,45 (2H), 7,99 (2H) ppm.

### Beispiel 52a

### 4-[4-((5R,8S,11R,13S,14S,17S)-5,17-Dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyloxycarbonylamino]-benzoesäure ethylester

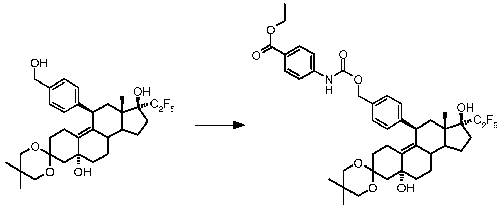

In Analogie zu Beispiel 32a setzte man 40 mg (67 µmol) der nach Beispiel 17d dargestellten Verbindung unter Verwendung von 4-Isocyanatbenzoesäureethylester um und setzte die nach Aufarbeitung erhaltene Titelverbindung als Rohprodukt weiter um.

### Beispiel 53

### [4-(3,5-Dimethyl-isoxazol-4-yl)-phenyl]-carbaminsäure 4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzylester

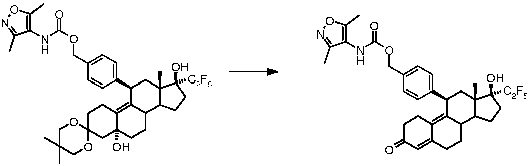

In Analogie zu Beispiel 1 setzte man das in Beispiel 53a dargestellte Rohprodukt um und isolierte nach Aufarbeitung und Reinigung 8,6 mg (20%) der Titelverbindung als farblosen Schaum.
¹H-NMR (CDCl₃): δ= 0,59 (3H), 1,39-1,57 (2H), 1,72-1,88 (3H), 1,99-2,65 (17H), 2,73 (1H), 4,45 (1H), 5,13 (2H), 5,79 (1H), 5,86 (1H), 7,19 (2H), 7,28 (2H) ppm.

### Beispiel 53a

### [4-(3,5-Dimethyl-isoxazol-4-yl)-phenyl]-carbaminsäure 4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzylester

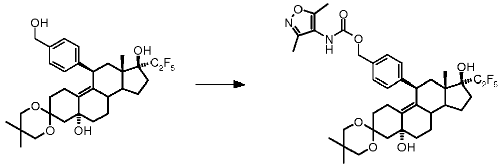

In Analogie zu Beispiel 32a setzte man 40 mg (67 µmol) der nach Beispiel 17d dargestellten Verbindung unter Verwendung von 3,5-Dimethylisoxazol-4-yl-isocyanat um und setzte die nach Aufarbeitung erhaltene Titelverbindung als Rohprodukt weiter um.

### Beispiel 55

### 3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyloxycarbonylamino]-propionsäure

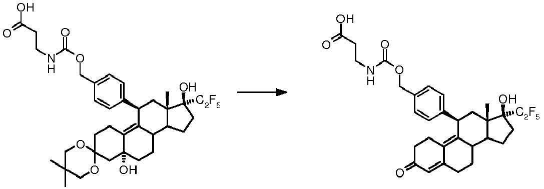

In Analogie zu Beispiel 1 setzte man 52 mg (73 µmol) der nach Beispiel 55a dargestellten Verbindung um und isolierte nach Aufarbeitung und Reinigung 14 mg (32%) der Titelverbindung als farblosen Schaum.
¹H-NMR (CD₃OD): δ= 0,57 (3H), 1,36-1,56 (2H), 1,70-1,82 (3H), 2,09 (1H), 2,16-2,46 (7H), 2,54-2,73 (4H), 2,79 (1H), 3,33 (2H), 4,52 (1H), 5,00 (2H), 5,73 (1H), 7,21 (2H), 7,26 (2H) ppm.

### Beispiel 55a

### 3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyloxycarbonylamino]-propionsäure

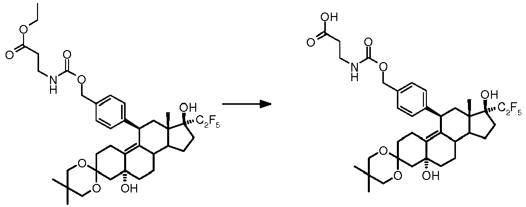

In Analogie zu Beispiel 26a setzte man 82 mg (0,11 mmol) der nach Beispiel 55b dargestellten Verbindung um und isolierte nach Aufarbeitung 59 mg (77%) der Titelverbindung als farblosen Schaum.

### Beispiel 55b

### 3-[4-((5R,8S,11R,13S,14S,17S)-5,17-dihydroxy-17-pentafluorethyl-5',5',13-trimethyl-1,2,3,4,6,7,8,11,12,13,14,15,16,17-tetradecahydro-spiro[cyclopenta[a]phenanthren-3,2'-[1,3]dioxan]-11-yl)-benzyloxycarbonylamino]-propionsäureethylester

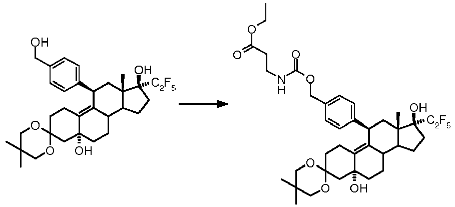

In Analogie zu Beispiel 32a setzte man 100 mg (0,17 mmol) der nach Beispiel 17d dargestellten Verbindung unter Verwendung von 3-Isocyanatopropionsäureethylester um und setzte die nach Aufarbeitung erhaltene Titelverbindung als Rohprodukt weiter um.

### Beispiel 56

### Benzyl-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester

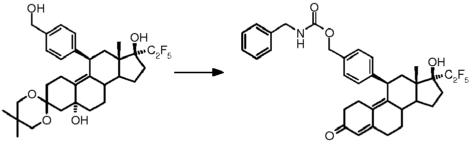

In Analogie zu Beispiel 32a setzte man 50 mg (83 µmol) der nach Beispiel 17d dargestellten Verbindung unter Verwendung von Benzylisocyanat um und setzte das nach Aufarbeitung erhaltene Rohprodukt in Analogie zu Beispiel 1 weiter um. Nach Aufarbeitung und Reinigung isolierte man 14 mg (27%) der Titelverbindung als farblosen Schaum.
¹H-NMR (CDCl₃): δ= 0,59 (3H), 1,42-1,55 (2H), 1,75-1,86 (3H), 2,06 (1H), 2,22-2,63 (10H), 2,72 (1H), 4,39 (2H), 4,45 (1H), 5,05 (1H), 5,09 (2H), 5,78 (1H), 7,17 (2H), 7,24-7,35 (7H) ppm.

### Beispiel 57

### 3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyloxycarbonylamino]-propionsäureethylester

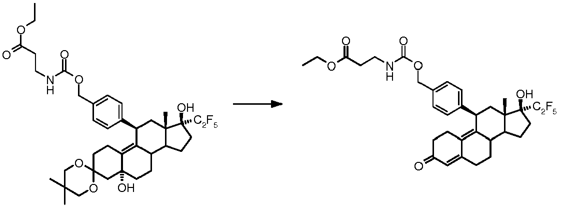

In Analogie zu Beispiel 1 setzte man 72 mg (97 µmol) der nach Beispiel 55b dargestellten Verbindung um und isolierte nach Aufarbeitung und Reinigung 6,1 mg (10%) der Titelverbindung als farblosen Schaum.
¹H-NMR (CDCl₃): δ= 0,59 (3H), 1,26 (3H), 1,42-1,56 (2H), 1,74-1,89 (3H), 2,07 (1H), 2,14 (1H), 2,22-2,67 (11H), 2,72 (1H), 3,47 (2H), 4,14 (2H), 4,45 (1H), 5,04 (2H), 5,28 (1H), 5,78 (1H), 7,16 (2H), 7,26 (2H) ppm.

### Beispiel 58

### Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MN-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0,01 nmol/l, 0,1 nmol/l, 1 nmol/l, 10 nmol/l, 100 nmol/l und 1 pmol/l) inkubiert, um die agonistische Wirksamkeit zu bestimmen. Als Positivkontrolle der Reportergeninduktion wurden die Zellen mit dem synthetischen Gestagen Promegeston (0,01 nmol/l, 0,1 nmol/l, 1 nmol/l, 10 nmol/l, 100 nmol/l and 1 pmol/l) behandelt. Zur Bestimmung der antagonistischen Aktivitat wurden die Zellen rnit 0,1 nmol/l Promegeston und zusätzlich mit steigenden Mengen der jeweiligen Testverbindung (0,01 nmol/l, 0,1 nmol/l, 1 nmol/l, 10 nmol/l, 100 nmol/l and 1 pmol/l) behandelt. Die Aktivität des Reportergens LUC (LUC = Luciferase) wurde in den Zellysaten bestimmt und als RLU (relative light units) gemessen. Alle Messwerte werden angegeben als % Wirksamkeit und als EC₅₀ bzw. IC₅₀ Konzentrationen.

### a) agonistische Aktivität:

Keine der genannten Verbindungen zeigt eine agonistische Aktivität.

### b) antagonistische Aktivität:

Alle genannten Verbindungen zeigen eine 100%ige antagonistische Wirksamkeit.

Die antagonistische Wirkstärke der Verbindungen ist in Tabelle 1 zusammengefasst.

**Tabelle 1: Antagonistische Wirkstärke der Verbindungen**

| Bsp. | PR-A IC₅₀ [nM] | PR-B IC₅₀ [nM] | Bsp. | PR-A IC₅₀ [nM] | PR-B IC₅₀ [nM] | Bsp. | PR-A IC₅₀ [nM] | PR-B IC₅₀ [nM] |
|---|---|---|---|---|---|---|---|---|
| | | | 19 | 0,6 | 0,7 | 39 | 0,1 | 0,2 |
| | | | 20 | 0,7 | 1,0 | | | |
| | | | 21 | 1,2 | 2,4 | 41 | 0,09 | 0,1 |
| | | | 22 | 0,5 | 0,6 | 42 | 0,1 | 0,2 |
| | | | 23 | 0,3 | 0,8 | 43 | 0,1 | 0,1 |
| | | | 24 | 0,4 | 0,5 | 44 | 0,1 | 0,1 |
| | | | | | | 45 | 0,02 | 0,09 |
| | | | 26 | 24 | 64 | 46 | 0,01 | 0,01 |
| | | | 27 | 43 | 29 | 47 | nb | nb |
| | | | | | | 48 | 0,07 | 0,2 |
| | | | | | | 49 | 0,09 | 0,1 |
| | | | | | | 50 | 0,1 | 0,4 |
| | | | | | | 51 | nb | nb |
| | | | 32 | 0,1 | 0,2 | 52 | 0,1 | 0,1 |
| | | | 33 | 0,8 | 0,8 | 53 | 0,09 | 0,03 |
| | | | 34 | 0,7 | 0,8 | | | |
| | | | 35 | 0,3 | 0,6 | 55 | 8 | 10 |
| | | | 36 | 0,7 | 0,8 | 56 | 0,2 | 0,3 |
| 17 | 0,4 | 0,8 | | | | 57 | 0,1 | 0,1 |
| 18 | 0,8 | 0,8 | 38 | 25 | 36 | | | |

| | | | | | | | | |
|---|---|---|---|---|---|---|---|---|
| nb: nicht bestimmt | | | | | | | | |

Bevorzugt sind Verbindungen, die eine antagonistische Wirkstärke mit einem IC₅₀ < 1,0 nM aufweisen.

Besonders bevorzugt sind Verbindungen, die eine antagonistische Wirkstärke mit einem IC₅₀ ≤ 0,1 nM aufweisen.

### Beispiel 59

### Bestimmung der metabolischen Stabilität in humanen und Ratten-Lebermikrosomen

Es wurden isolierte humane Lebermikrosomen (HLM) zur Beurteilung der metabolischen Stabilität von Verbindungen der allgemeinen Formel I eingesetzt.

Die Inkubationen wurden mit 2.4 ml HLM-lösung (0.5 mg/ml Proteingehalt), 30 µl der Testverbindung (finale Konzentration 1 µM) und 0.6 ml des Cofaktorgemisches (=NADPH-generierendem System aus 3 IU Glucose-6-phosphatdehydrogenase, 14.6 mg Glucose-6-phosphat, 1.2 mg NADP) bei 37°C in 100 mM Phosphatpuffer bei pH 7.4 durchgeführt. Es wurden zu 6 Zeitpunkten (2 - 60 min) Proben entnommen, mit gleichem Volumen Methanol gefällt und der Wiederfund der eingesetzten Testsubstanzen im Überstand mittels LC-MS/MS-Analytik ermittelt. Aus der daraus ermittelten Halbwertzeit des Substanzabbaus wurde die sogenannte intrinsische Clearance der Substanz im Lebermikrosomenansatz berechnet. Mit Hilfe dieser wurde unter Zuhilfenahme verschiedener physiologischer Kenngrößen (humaner Leberblutfluss: 1.3 L*kg/h; spezifisches Lebergewicht (pro kg Körpergewicht): 21g/kg; mikrosomaler Proteingehalt: 40mg/g Leber) nach dem well-stirred Modell eine (metabolische) *in vivo* Clearance in Bezug auf Phase I Reaktionen vorhersagt. Weiterhin wurde unter den Annahmen, dass (i) die Absorption der Prüfsubstanz 100% beträgt, und (ii) der First pass vollständig vom Lebermikrosomenmetabolismus abgebildet wird, eine maximale orale Bioverfügbarkeit (Fmax) errechnet.

Einige der getesteten Verbindungen weisen eine für den Menschen vorhergesagte orale Bioverfügbarkeit von Fmax < 50% auf, weshalb sie sich besonders gut für lokalen Anwendungen, die eine reduzierte systemische Stabilität erfordern, eignen. Dazu gehören beispielsweise die in den Beispielen 17, 23, 24 und 41 genannten Verbindungen (siehe Tabelle 2)

**Tabelle 2**

| Beispiel | vorhergesagte Clᵢₙₜ [l/h/kg] | | vorhergesagte Fₘₐₓ [%] (maximale orale Bioverfügbarkeit) | |
|---|---|---|---|---|
| | rat | human | rat | human |
| 17 | 1,6 | 1,17 | 63 | 12 |
| 23 | 3,0 | 1,27 | 29 | 4 |
| 24 | 1,0 | 1,08 | 77 | 18 |
| 41 | 2,1 | 1,17 | 49 | 11 |

Ganz besonders bevorzugt für systemische Anwendungen sind solche Verbindungen, die eine vorhergesagte maximale Bioverfügbarkeit bei unterschiedlichen Spezies (Ratte, Mensch) aufweisen, die jeweils größer 60% ist.

Ganz besonders bevorzugt für lokale Anwendungen sind solche Verbindungen, die eine vorhergesagte maximale Bioverfügbarkeit für den Menschen aufweisen, die kleiner 50% ist.

## Patentansprüche

1. Verbindung der Formel (II) 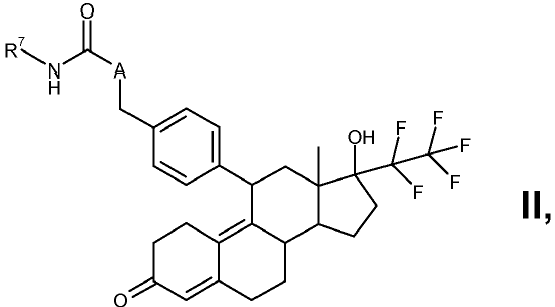 worin
A entweder -O- oder -NH- und
R⁷ C₁-C₄-Alkyl-, Allyl-, gegebenenfalls in para-Postion durch die Gruppen C₁-C₄-Alkyl, -CN, C₁-C₄-Alkoxy, Halogen, C₁-C₄-Alkanoyloxy, -CO₂H, -CO₂C₁₋₄-Alkyl oder Piperidinyl, substituiertes Phenyl-, 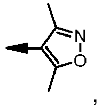 Benzyl-, -(CH₂)ₘ-COOR⁸ mit m = 1, 2 oder 3 und R⁸ = Wasserstoff oder C₁-C₄-Alkyl
bedeutet und ihre Salze, Solvate oder Solvate der Salze, einschließlich aller Kristallmodifikationen, die α-, β- oder γ-Cyclodextrinclathrate, sowie die mit Liposomen verkapselten Verbindungen, wobei Phenyl kann einfach oder mehrfach mit Halogen (F, Cl, Br, I), OH, O-Alkyl, CO₂H, CO₂-Alkyl, NH₂, NH(C₁-C₁₀)-Alkyl), N(C₁-C₁₀-Alkyl)₂, NO₂, N₃, C₁-C₁₀-Alkyl, C₁-C₁₀-Perfluor-Alkyl, C₁-C₁₀-Acyl oder C₁-C₁₀-Acyloxy-Gruppen substituiert sein.

2. Verbindung der Formel (III) 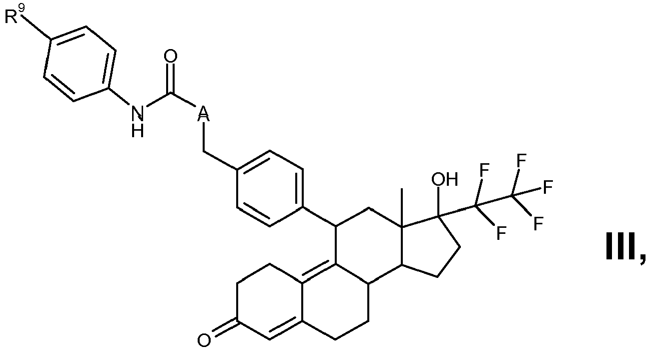 worin
A entweder -O- oder -NH- und
R⁹ Wasserstoff, C₁-C₄-Alkyl, -CN, C₁-C₄-Alkoxy, Halogen, C₁-C₄-Alkanoyloxy,-CO₂H, oder -CO₂C₁-₄-Alkyl
bedeutet und ihre Salze, Solvate oder Solvate der Salze, einschließlich aller Kristallmodifikationen, die α-, β- oder γ-Cyclodextrinclathrate, sowie die mit Liposomen verkapselten Verbindungen.

3. Verbindungen, nämlich
1-Ethyl-3-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-harnstoff (Beispiel 17)
1-Allyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 18)
1-Isopropyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 19)
1-*tert*-Butyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzyl]-harnstoff (Beispiel 20)
{3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyl]-ureido}-essigsäureethylester (Beispiel 21)
3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-ureido}-propionsäureethylester (Beispiel 22)
1-Benzyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 23)
1-Phenyl-3-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 24)
{3-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-ureido}-essigsäure (Beispiel 26)
3-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-ureido}-propionsäure (Beispiel 27)
1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-3-(4-methoxy-phenyl)-harnstoff (Beispiel 32)
1-(4-Fluor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 33)
1-(4-Chlor-phenyl)-3-[4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-harnstoff (Beispiel 34)
1-(4-*tert*-Butyl-phenyl)-3-[4-((8S,11R,13S,14S, 17S)-17-hydroxy-13-methyl-3-oxo-17-pentafluorethyl-2,3,6,7,8,11,12,13,14,15, 16,17-dodecahydro-1*H-*cyclopenta[*a*]phenanthren-11-yl)-benzyl]-harnstoff (Beispiel 35)
4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-ureido}-benzoesäure ethylester (Beispiel 36)
4-{3-[4-((8S,11R,13S,14S,17S)-17-Hydroxy-13-methyl-3-oxo-17-pentafluorethyl-2,3,6,7,8,11,12,13,14,15,16,17-dodecahydro-1*H-*cyclopenta[*a*]phenanthren-11-yl)-benzyl]-ureido}-benzoesäure (Beispiel 38)
1-[4-((8S,11R,13S,14S,17S)-17-Hydroxy-13-methyl-3-oxo-17-pentafluorethyl-2,3,6,7,8,11,12,13,14,15,16,17-dodecahydro-1H-cyclopenta[a]phenanthren-11-yl)-benzyl]-3-(4-piperidin-1-yl-phenyl)-harnstoff (Beispiel 39)
Ethyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzylester (Beispiel 41)
Isopropyl-carbaminsäure 4-((8S,11R,13S,14S, 17S)-17-hydroxy-13-methyl-3-oxo-17-pentafluorethyl-2,3,6,7,8,11,12,13,14,15, 16,17-dodecahydro-1*H-*cyclopenta[*a*]phenanthren-11-yl)-benzylester (Beispiel 42)
Allyl-carbaminsäure 4-((8S,11R,13S,14S, 17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-benzylester (Beispiel 43)
*tert*-Butyl-carbaminsäure 4-((8S,11R,13S,14S, 17S)-17-hydroxy-13-methyl-3-oxo-17-pentafluorethyl-2,3,6,7,8,11,12,13,14,15,16, 17-dodecahydro-1*H-*cyclopenta[*a*]phenanthren-11-yl)-benzylester (Beispiel 44)
(4-Piperidin-1-yl-phenyl)-carbaminsäure 4-((8S,11R,13S,14S,17S)-17-hydroxy-13-methyl-3-oxo-17-pentafluorethyl-2,3,6,7,8,11,12,13, 14,15,16,17-*dodecahydro*-1*H-*cyclopenta[*a*] phenanthren-11-yl)-benzylester (Beispiel 45)
Phenyl-carbaminsäure 4-((8S,11R,13S,14S, 17S)-17-hydroxy-13-methyl-3-oxo-17-pentafluorethyl-2,3,6,7,8,11,12,13,14,15,16,17-*dodecahydro*-1*H-*cyclopenta[a] phenanthren-11-yl)-benzylester (Beispiel 46)
(4-Methoxy-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester (Beispiel 47)
(4-Methyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester (Beispiel 48)
(4-Fluor-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester (Beispiel 49)
(4-Chlor-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester (Beispiel 50)
(4-*tert*-Butyl-phenyl)-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1H-cyclopenta[a] phenanthren-11-yl)-benzylester (Beispiel 51)
4-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyloxycarbonylamino]-benzoesäure ethylester (Beispiel 52)
[4-(3,5-Dimethyl-isoxazol-4-yl)-phenyl]-carbaminsäure 4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzylester (Beispiel 53)
3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[a]phenanthren-11-yl)-benzyloxycarbonylamino]-propionsäure (Beispiel 55)
Benzyl-carbaminsäure 4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[*a*] phenanthren-11-yl)-benzylester (Beispiel 56)
3-[4-((8S,11R,13S,14S,17S)-17-Hydroxy-13-methyl-3-oxo-17-pentafluorethyl-2,3,6,7,8,11,12,13,14,15,16,17-dodecahydro-1*H*-cyclopenta[*a*]phenanthren-11-yl)-benzyloxycarbonylamino]-propionsäureethylester (Beispiel 57).

4. Verbindungen nach einem der Ansprüche 1 - 3, die eine Progesteronerezeptorantagonistische Wirkung in stabilen Transfektanten humaner Neuroblastoma-Zellen zeigen.

5. Eine Verbindungen gemäß einem der vorstehenden Ansprüche zur Behandlung und Prophylaxe von Krankheiten.

6. Eine Verbindung gemäß einem der Ansprüche 1 - 3 zur Behandlung und Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

7. Verwendung einer Verbindung nach einem der Ansprüche 1 - 3 zur Herstellung eines Arzneimittels zur Behandlung und/oder Prophylaxe von Krankheiten.

8. Verwendung einer Verbindung nach einem der Ansprüche 1 - 3 zur Herstellung eines Arzneimittels zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

9. Arzneimittel enthaltend eine Verbindung, wie in einem der Ansprüche 1 - 3 definiert, in Kombination mit einem weiteren Wirkstoff.

10. Arzneimittel enthaltend eine Verbindung, wie in einem der Ansprüche 1 - 3 definiert, in Kombination mit einem inerten, nichttoxischen, pharmazeutisch geeigneten Hilfsstoff.

11. Arzneimittel nach Anspruch 9 oder 10 zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

## Claims

1. Compound of the formula (II) 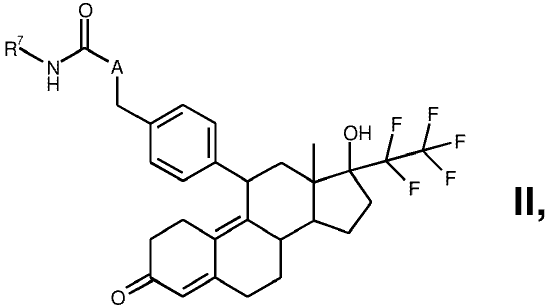 in which
A is either -O- or -NH- and
R⁷ is C₁-C₄-alkyl, allyl, phenyl optionally substituted in the para-position by the C₁-C₄-alkyl, -CN, C₁-C₄-alkoxy, halogen, C₁-C₄-alkanoyloxy, -CO₂H, -CO₂C₁₋₄-alkyl or piperidinyl groups, 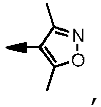 benzyl, - (CH₂)ₘ-COOR⁸ where m = 1, 2 or 3 and R⁸ = hydrogen or C₁-C₄-alkyl
and the salts, solvates or solvates of the salts thereof, including all crystal polymorphs, the α-, β- or γ-cyclodextrin clathrates, and the compounds encapsulated with liposomes, where phenyl may be mono- or polysubstituted by halogen (F, Cl, Br, I), OH, O-alkyl, CO₂H, CO₂-alkyl, NH₂, NH(C₁-C₁₀)-alkyl), N(C₁-C₁₀-alkyl)₂, NO₂, N₃, C₁-C₁₀-alkyl, C₁-C₁₀-perfluoroalkyl, C₁-C₁₀-acyl or C₁-C₁₀-acyloxy groups.

2. Compound of the formula (III) 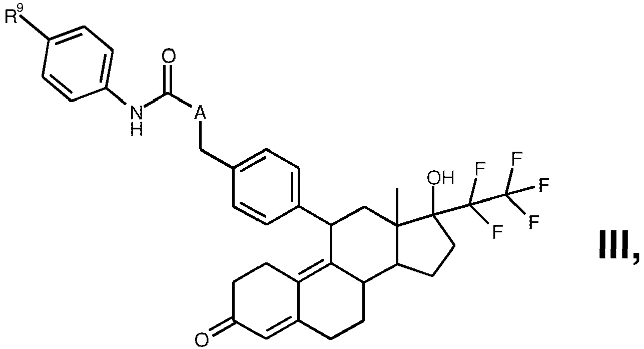 in which
A is either -O- or -NH- and
R⁹ is hydrogen, C₁-C₄-alkyl, -CN, C₁-C₄-alkoxy, halogen, C₁-C₄-alkanoyloxy, -CO₂H, or -CO₂C₁₋₄-alkyl
and the salts, solvates or solvates of the salts thereof, including all crystal polymorphs, the α-, β- or γ-cyclodextrin clathrates, and the compounds encapsulated with liposomes.

3. Compounds, specifically
1-ethyl-3-[4-((8S,11R,13S,14S,17S)-17-hydroxy-13-methyl-3-oxo-17-pentafluoroethyl-2,3,6,7,8,11,12,13,14,15,16,17-dodecahydro-1H-cyclopenta[a]phenanthren-11-yl)benzyl]urea (Example 17)
1-allyl-3-[4-((8S,11R,135,145,17S)-17-hydroxy-13-methyl-3-oxo-17-pentafluoroethyl-2,3,6,7,8,11,12,13,14,15,16,17-dodecahydro-*1H-*cyclopenta[a]phenanthren-11-yl)benzyl]urea (Example 18)
1-isopropyl-3-[4-((8S,11R,13S,14S,17S)-17-hydroxy-13-methyl-3-oxo-17-pentafluoroethyl-2,3,6,7,8,11,12,13,14,15,16,17-dodecahydro-*1H-*cyclopenta[a]phenanthren-11-yl)benzyl]urea (Example 19)
1-*tert*-butyl-3-[4-((8S,11R,13S,14S,17S)-17-hydroxy-13-methyl-3-oxo-17-pentafluoroethyl-2,3,6,7,8,11,12,13,14,15,16,17-dodecahydro-*1H-*cyclopenta[a]phenanthren-11-yl)benzyl]urea (Example 20)
{3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}acetic acid ethyl ester (Example 21)
3-{3-[4-((8S,11R,135,145,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}propionic acid ethyl ester (Example 22)
1-benzyl-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea (Example 23)
1-phenyl-3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea (Example 24)
{3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}acetic acid (Example 26)
3-{3-[4-((8S,11R,135,145,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}propionic acid (Example 27)
1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]-3-(4-methoxyphenyl)urea (Example 32)
1-(4-fluorophenyl)-3-[4-((8S,11R,135,145, 17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea (Example 33)
1-(4-chlorophenyl)-3-[4-((8S,11R,13S,14S, 17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]urea (Example 34)
1-(4-*tert*-butylphenyl)-3-[4-((8S,11R,13S,14S, 17S)-17-hydroxy-13-methyl-3-oxo-17-pentafluoroethyl-2,3,6,7,8,11,12,13,14,15, 16,17-dodecahydro-1*H*-cyclopenta[*a*]phenanthren-11-yl)benzyl]urea (Example 35)
4-{3-[4-((8S,11R,135,145,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}benzoic acid ethyl ester (Example 36)
4-{3-[4-((8S,11R,135,145,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]ureido}benzoic acid (Example 38)
1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl]-3-(4-piperidin-1-ylphenyl)urea (Example 39)
ethylcarbamic acid 4-((8S,11R,13S,14S, 17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl ester (Example 41)
isopropylcarbamic acid 4-((8S,11R,13S,14S, 17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl ester (Example 42)
allylcarbamic acid 4-((8S,11R,13S,14S, 17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl ester (Example 43)
*tert*-butylcarbamic acid 4-((8S,11R,13S,14S, 17S)-17-hydroxy-13-methyl-3-oxo-17-pentafluoroethyl-2,3,6,7,8,11,12,13,14,15,16, 17-dodecahydro-1*H-*cyclopenta[*a*]phenanthren-11-yl)benzyl ester (Example 44)
(4-piperidin-1-ylphenyl)carbamic acid 4-((8S,11R,13S,14S,17S)-17-hydroxy-13-methyl-3-oxo-17-pentafluoroethyl-2,3,6,7,8,11,12,13, 14,15,16*,*17-*dodecahydro-*1*H-*cyclopenta[*a*]phenanthren-11-yl)benzyl ester (Example 45)
phenylcarbamic acid 4-((8S,11R,13S,14S, 17S)-17-hydroxy-13-methyl-3-oxo-17-pentafluoroethyl-2,3,6,7,8,11,12,13,14,15,16,17-*dodecahydro*-1*H-*cyclopenta[*a*]phenanthren-11-yl)benzyl ester (Example 46)
(4-methoxyphenyl)carbamic acid 4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)benzyl ester (Example 47)
(4-methylphenyl)carbamic acid 4-((8S,11R,135,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)benzyl ester (Example 48)
(4-fluorophenyl)carbamic acid 4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)benzyl ester (Example 49)
(4-chlorophenyl)carbamic acid 4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)benzyl ester (Example 50)
(4-tert-butylphenyl)carbamic acid 4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)benzyl ester (Example 51)
4-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyloxycarbonylamino]benzoic acid ethyl ester (Example 52)
[4-(3,5-dimethylisoxazol-4-yl)phenyl]carbamic acid 4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyl ester (Example 53)
3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyloxycarbonylamino]propionic acid (Example 55)
benzylcarbamic acid 4-((8S,11R,13S,14S,17S)-17-hydroxy-13-methyl-3-oxo-17-pentafluoroethyl-2,3,6,7,8,11,12,13,14, 15,16,17-dodecahydro-1*H-*cyclopenta[*a*]phenanthren-11-yl)benzyl ester (Example 56)
3-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)benzyloxycarbonylamino]propionic acid ethyl ester (Example 57).

4. Compounds according to any of Claims 1 - 3 which exhibit progesterone receptor-antagonistic action in stable transfectants of human neuroblastoma cells.

5. A compound according to any of the preceding claims for treatment and prophylaxis of disorders.

6. A compound according to any of Claims 1 - 3 for treatment and prophylaxis of fibroids of the uterus, endometriosis, heavy menstrual bleeds, meningiomas, hormone-dependent breast cancers and complaints associated with the menopause, or for fertility control and emergency contraception.

7. Use of a compound according to any of Claims 1 - 3 for producing a medicament for treatment and/or prophylaxis of disorders.

8. Use of a compound according to any of Claims 1 - 3 for producing a medicament for treatment and/or prophylaxis of fibroids of the uterus, endometriosis, heavy menstrual bleeds, meningiomas, hormone-dependent breast cancers and complaints associated with the menopause, or for fertility control and emergency contraception.

9. Medicament comprising a compound as defined in any of Claims 1 - 3 in combination with a further active ingredient.

10. Medicament comprising a compound as defined in any of Claims 1 - 3 in combination with an inert, nontoxic, pharmaceutically suitable excipient.

11. Medicament according to Claim 9 or 10 for treatment and/or prophylaxis of fibroids of the uterus, endometriosis, heavy menstrual bleeds, meningiomas, hormone-dependent breast cancers and complaints associated with the menopause, or for fertility control and emergency contraception.

## Revendications

1. Composé de formule (II) 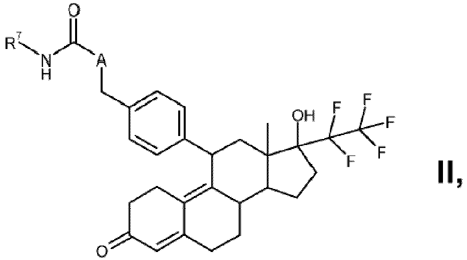 dans laquelle
A signifie -O- ou -NH- et
R⁷ signifie alkyle en C₁-C₄, allyle, phényle éventuellement substitué en position para par les groupes alkyle en C₁-C₄, -CN, alcoxy en C₁-C₄, halogène, alcanoyloxy en C₁-C₄, -CO₂H, -CO₂-alkyle en C₁-C₄ ou pipéridinyle ; 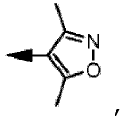 benzyle, - (CH₂)ₘ-COOR⁸ avec m = 1, 2 ou 3, et R⁸ = hydrogène ou alkyle en C₁-C₄,
et ses sels, solvates ou solvates des sels, y compris toutes les formes cristallines, les clathrates d'α-, β- ou γ-cyclodextrine, ainsi que les composés encapsulés avec des liposomes, le phényle pouvant être substitué une ou plusieurs fois avec des groupes halogène (F, Cl, Br, I), OH, O-alkyle, CO₂H, CO₂-alkyle, NH₂, NH(alkyle en C₁-C₁₀), N(alkyle en C₁-C₁₀)₂, NO₂, N₃, alkyle en C₁-C₁₀, perfluoroalkyle en C₁-C₁₀, acyle en C₁-C₁₀ ou acyloxy en C₁-C₁₀.

2. Composé de formule (III) 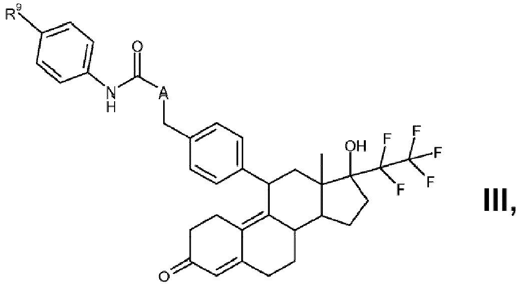 dans laquelle
A signifie -O- ou -NH-, et
R⁹ signifie hydrogène, alkyle en C₁-C₄, -CN, alcoxy en C₁-C₄, halogène, alcanoyloxy en C₁-C₄, -CO₂H ou -CO₂-alkyle en C₁-C₄,
et ses sels, solvates ou solvates des sels, y compris toutes les formes cristallines, les clathrates d'α-, β- ou γ-cyclodextrine, ainsi que les composés encapsulés avec des liposomes.

3. Composés, à savoir :
1-éthyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 17)
1-allyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 18)
1-isopropyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 19)
1-tert-butyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 20)
ester éthylique de l'acide {3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-acétique (Exemple 21)
ester éthylique de l'acide 3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-propionique (Exemple 22)
1-benzyl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 23)
1-phényl-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 24)
acide {3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-acétique (Exemple 26)
acide 3-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-propionique (Exemple 27)
1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-3-(4-méthoxy-phényl)-urée (Exemple 32)
1-(4-fluoro-phényl)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 33)
1-(4-chloro-phényl)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 34)
1-(4-tert-butyl-phényl)-3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-urée (Exemple 35)
ester éthylique de l'acide 4-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-benzoïque (Exemple 36)
acide 4-{3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-uréido}-benzoïque (Exemple 38)
1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyl]-3-(4-pipéridin-1-yl-phényl)-urée (Exemple 39)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide éthyl-carbamique (Exemple 41)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide isopropyl-carbamique (Exemple 42)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide allyl-carbamique (Exemple 43)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide tert-butylcarbamique (Exemple 44)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-pipéridin-1-yl-phényl)-carbamique (Exemple 45)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide phényl-carbamique (Exemple 46)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-méthoxy-phényl)-carbamique (Exemple 47)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-méthyl-phényl)-carbamique (Exemple 48)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-fluoro-phényl)-carbamique (Exemple 49)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-chloro-phényl)-carbamique (Exemple 50)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide (4-tert-butyl-phényl)-carbamique (Exemple 51)
ester éthylique de l'acide 4-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyloxycarbonylaminol-benzoïque (Exemple 52)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide [4-(3,5-diméthyl-isoxazol-4-yl)-phényl]-carbamique (Exemple 53)
acide 3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyloxycarbonylamino]-propionique (Exemple 55)
ester 4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzylique de l'acide benzylcarbamique (Exemple 56)
ester éthylique de l'acide 3-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-benzyloxycarbonylamino]-propionique (Exemple 57).

4. Composés selon l'une quelconque des revendications 1 à 3, qui présentent un effet antagoniste des récepteurs de progestérone dans des transfectants stables de cellules humaines de neuroblastome.

5. Composés selon l'une quelconque des revendications précédentes pour le traitement et la prophylaxie de maladies.

6. Composé selon l'une quelconque des revendications 1 à 3 pour le traitement et la prophylaxie de fibroïdes utérins, de l'endométriose, des menstruations fortes, des méningiomes, des cancers du sein hormonodépendants et des troubles associés à la ménopause ou pour le contrôle de la fertilité et la contraception d'urgence.

7. Utilisation d'un composé selon l'une quelconque des revendications 1 à 3 pour la fabrication d'un médicament pour le traitement et/ou la prophylaxie de maladies.

8. Utilisation d'un composé selon l'une quelconque des revendications 1 à 3 pour la fabrication d'un médicament pour le traitement et/ou la prophylaxie de fibroïdes utérins, de l'endométriose, des menstruations fortes, des méningiomes, des cancers du sein hormonodépendants et des troubles associés à la ménopause ou pour le contrôle de la fertilité et la contraception d'urgence.

9. Médicament contenant un composé tel que défini dans l'une quelconque des revendications 1 à 3 en combinaison avec un autre agent actif.

10. Médicament contenant un composé tel que défini dans l'une quelconque des revendications 1 à 3 en combinaison avec un adjuvant inerte, non toxique, pharmaceutiquement approprié.

11. Médicament selon la revendication 9 ou 10 pour le traitement et/ou la prophylaxie de fibroïdes utérins, de l'endométriose, des menstruations fortes, des méningiomes, des cancers du sein hormonodépendants et des troubles associés à la ménopause ou pour le contrôle de la fertilité et la contraception d'urgence.
